(19) **Europäisches Patentamt / European Patent Office / Office européen des brevets**

(11) **EP 3 218 100 B1**

(12) **FASCICULE DE BREVET EUROPEEN**

(45) Date de publication et mention de la délivrance du brevet:
**24.03.2021 Bulletin 2021/12**

(51) Int Cl.:
*B01J 20/18* (2006.01)  *B01J 20/30* (2006.01)
*B01J 20/28* (2006.01)  *C07C 7/13* (2006.01)
*C07C 29/76* (2006.01)  *C07C 37/82* (2006.01)

(21) Numéro de dépôt: **15797923.8**

(22) Date de dépôt: **13.11.2015**

(86) Numéro de dépôt international:
**PCT/EP2015/076531**

(87) Numéro de publication internationale:
**WO 2016/075280 (19.05.2016 Gazette 2016/20)**

(54) **ADSORBANTS ZÉOLITHIQUES À BASE DE ZÉOLITHE X À FAIBLE TAUX DE LIANT ET À FAIBLE SURFACE EXTERNE, LEUR PROCÉDÉ DE PRÉPARATION ET LEURS UTILISATIONS**

**ZEOLITHADSORPTIONSMITTEL AUS X-ZEOLITH MIT GERINGEM BINDEMITTELGEHALT UND GERINGEM EXTERNEM OBERFLÄCHENBEREICH, VERFAHREN ZUR HERSTELLUNG DAVON UND VERWENDUNGEN DAVON**

**ZEOLITE ADSORBENTS MADE FROM X ZEOLITE WITH LOW BINDER CONTENT AND LOW EXTERNAL SURFACE AREA, METHOD FOR PREPARATION OF SAME AND USES THEREOF**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.11.2014 FR 1460955**

(43) Date de publication de la demande:
**20.09.2017 Bulletin 2017/38**

(73) Titulaires:
• **IFP Energies nouvelles**
  **92500 Rueil-Malmaison (FR)**
• **ARKEMA FRANCE**
  **92700 Colombes (FR)**

(72) Inventeurs:
• **BOUVIER, Ludivine**
  **F-64300 Orthez (FR)**
• **LUTZ, Cécile**
  **F-64290 Gan (FR)**
• **LAROCHE, Catherine**
  **F-69390 Charly (FR)**

(74) Mandataire: **Bandpay & Greuter**
  **30, rue Notre-Dame des Victoires**
  **75002 Paris (FR)**

(56) Documents cités:
  **EP-A2- 1 142 622    WO-A1-2008/009845**

WO-A1-2014/090771    FR-A1- 2 789 914
US-A1- 2012 093 715    US-A1- 2012 247 334
US-A1- 2014 086 817

• **ZHONGLIN LI ET AL: "Preparation of granular X-type zeolite/activated carbon composite from elutrilithe by adding pitch and solid SiO2", MATERIALS CHEMISTRY AND PHYSICS, vol. 147, no. 3, 15 juillet 2014 (2014-07-15), pages 1003-1008, XP055197875, ISSN: 0254-0584, DOI: 10.1016/j.matchemphys.2014.06.051**
• **NINA-LUISA MICHELS ET AL: "Hierarchically Structured Zeolite Bodies: Assembling Micro-, Meso-, and Macroporosity Levels in Complex Materials with Enhanced Properties", ADVANCED FUNCTIONAL MATERIALS, WILEY - V C H VERLAG GMBH & CO. KGAA, DE, vol. 22, no. 12, 20 juin 2012 (2012-06-20) , pages 2509-2518, XP001576540, ISSN: 1616-301X, DOI: 10.1002/ADFM.201103120 [extrait le 2012-03-21]**
• **ALEXANDRA INAYAT ET AL: "Assemblies of Mesoporous FAU-Type Zeolite Nanosheets", ANGEWANDTE CHEMIE INTERNATIONAL EDITION, vol. 51, no. 8, 20 février 2012 (2012-02-20), pages 1962-1965, XP055116858, ISSN: 1433-7851, DOI: 10.1002/anie.201105738 cité dans la demande**

EP 3 218 100 B1

- **DANNY VERBOEKEND ET AL: "Hierarchical FAU- and LTA-Type Zeolites by Post-Synthetic Design: A New Generation of Highly Efficient Base Catalysts", ADVANCED FUNCTIONAL MATERIALS, vol. 23, no. 15, 19 avril 2013 (2013-04-19), pages 1923-1934, XP055117151, ISSN: 1616-301X, DOI: 10.1002/adfm.201202320**

**Description**

L'invention concerne des adsorbants zéolithiques sous forme d'agglomérés à faible taux de liant comprenant de la zéolithe faujasite pour leurs utilisations dans des applications où le transfert de matière est un paramètre important, lesdits adsorbants présentant une faible surface externe inférieure ou égale à 30 m$^2$.g$^{-1}$, de préférence inférieure ou égale à 20 m$^2$.g$^{-1}$.

La présente invention concerne également un procédé de préparation desdits adsorbants zéolithiques, ainsi que leurs utilisations, notamment pour la séparation de mélanges gazeux ou liquides d'isomères, plus particulièrement des xylènes et notamment pour la production de paraxylène très pur à partir d'une charge d'hydrocarbures aromatiques contenant des isomères à 8 atomes de carbone.

L'utilisation d'adsorbants zéolithiques comprenant au moins de la zéolithe Faujasite (FAU) de type X et comprenant, outre des cations sodium, des ions baryum, potassium ou strontium, seuls ou en mélanges, pour adsorber sélectivement le paraxylène dans un mélange d'hydrocarbures aromatiques, est bien connue de l'art antérieur.

Les brevets US 3 558 730, US 3 558 732, US 3 626 020 et US 3 663 638 montrent que des adsorbants zéolithiques comprenant des aluminosilicates à base de sodium et de baryum (US 3 960 774) ou à base de sodium, de baryum et de potassium, sont efficaces pour la séparation du paraxylène présent dans des coupes aromatiques en C8 (coupes comprenant des hydrocarbures aromatiques à 8 atomes de carbone).

Dans les brevets listés ci-dessus, les adsorbants zéolithiques se présentent sous forme de cristaux à l'état de poudre ou sous forme d'agglomérés constitués majoritairement de poudre de zéolithe et jusqu'à 20% en poids de liant inerte. La publication scientifique de Zhonglin Li et al, dans la revue Materials Chemistry and Physics, (15 juillet 2014), vol. 147, no 3, pages 1003-1008, intitulée «Préparation of granular X-type zeolite/activated carbon composite from elutrilithe by adding pitch and solid SiO2 » divulgue un procédé de synthèse d'un matériau composite à base de zéolithe de type X et de carbone activé. Des variations dans le procédé de synthèse ont été étudiées.

La synthèse des zéolithes FAU s'effectue habituellement par nucléation et cristallisation de gels de silico-aluminates. Cette synthèse conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, atomisation et autres techniques d'agglomération connues de l'homme du métier. Ces agglomérés ne présentent pas les inconvénients inhérents aux matières pulvérulentes.

Les agglomérés, qu'ils soient sous forme de plaquettes, de billes, d'extrudés, et autres, sont en général constitués de cristaux de zéolithe(s), qui constituent l'élément actif (au sens de l'adsorption) et d'un liant d'agglomération. Ce liant d'agglomération est destiné à assurer la cohésion des cristaux entre eux dans la structure agglomérée, mais aussi doit permettre d'assurer une résistance mécanique suffisante auxdits agglomérés afin d'éviter, ou tout au moins de minimiser le plus possible, les risques de fractures, brisures ou cassures qui pourraient survenir lors de leurs utilisations industrielles pendant lesquelles les agglomérés sont soumis à de nombreuses contraintes, telles que vibrations, variations fortes et/ou fréquentes de pressions, mouvements et autres.

La préparation de ces agglomérés s'opère par exemple par empâtage de cristaux de zéolithe à l'état de poudre avec une pâte argileuse, dans des proportions de l'ordre de 80% à 90% en poids de poudre de zéolithe pour 20% à 10% en poids de liant, puis mise en forme en billes, plaquettes ou extrudés, et traitement thermique à haute température pour cuisson de l'argile et réactivation de la zéolithe, le ou les échange(s) cationique(s), comme par exemple l'échange au baryum et éventuellement au potassium pouvant être effectué avant et/ou après l'agglomération de la zéolithe pulvérulente avec le liant.

On obtient des corps zéolithiques dont la granulométrie est de quelques millimètres, voire de l'ordre du millimètre, et qui, si le choix du liant d'agglomération et la granulation sont faits dans les règles de l'art, présentent un ensemble de propriétés satisfaisantes, en particulier de porosité, de résistance mécanique, de résistance à l'abrasion. Cependant, les propriétés d'adsorption de ces agglomérés sont évidemment réduites par rapport à la poudre active de départ en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer facilement cette opération, on utilise des liants zéolithisables, le plus souvent appartenant à la famille de la kaolinite, et de préférence préalablement calcinés à des températures généralement comprises entre 500°C et 700°C.

Le brevet FR 2 789 914 décrit un procédé de fabrication d'agglomérés de zéolithe X, de rapport atomique Si/Al compris entre 1,15 et 1,5, échangés au baryum et éventuellement au potassium, en agglomérant des cristaux de zéolithe X avec un liant, une source de silice et de la carboxyméthylcellulose, puis en zéolithisant le liant par immersion de l'aggloméré dans une liqueur alcaline. Après échange des cations de la zéolithe par des ions baryum (et éventuellement potassium) et activation, les agglomérés ainsi obtenus présentent des résultats de sélectivité d'adsorption du paraxylène vis-à-vis des autres molécules aromatiques en C8 identiques et une augmentation de la capacité d'adsorption du paraxylène, par rapport à des adsorbants préparés à partir de la même quantité de zéolithe X et de liant, mais dont le liant n'est pas zéolithisé. Le brevet FR 2 789 914 enseigne ainsi que la zéolithisation du liant permet une augmentation de la capacité d'adsorption du paraxylène, sans modification des propriétés de sélectivité d'adsorption.

Outre une capacité d'adsorption élevée et de bonnes propriétés de sélectivité vis-à-vis de l'espèce à séparer du mélange réactionnel, l'adsorbant doit présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (*ibid.,* page 243), que, dans le cas d'un adsorbant aggloméré, le transfert de matière global dépend de la somme des résistances diffusionnelles intra-cristalline et inter-cristalline (entre les cristaux).

La résistance diffusionnelle intra-cristalline est proportionnelle au carré des diamètres des cristaux et inversement proportionnelle à la diffusivité intracristalline des molécules à séparer. La résistance diffusionnelle inter-cristalline (également appelée « résistance macroporeuse ») est quant à elle proportionnelle au carré des diamètres des agglomérés, inversement proportionnelle à la porosité contenue dans les macropores et mésopores (c'est-à-dire les pores dont l'ouverture est supérieure à 2 nm) au sein de l'aggloméré, et inversement proportionnelle à la diffusivité des molécules à séparer dans cette porosité.

La taille des agglomérés est un paramètre important lors de l'utilisation de l'adsorbant dans l'application industrielle, car elle détermine la perte de charge au sein de l'unité industrielle et l'uniformité du remplissage. La distribution granulométrique des agglomérés doit donc être étroite, et centrée sur des diamètres moyens en nombre compris typiquement entre 0,40 mm et 0,65 mm afin d'éviter des pertes de charges excessives.

La porosité contenue dans les macropores et mésopores au sein de l'aggloméré (respectivement macroporosité et mésoporosité inter-cristallines) peut être augmentée en utilisant des agents porogènes, tels que par exemple l'amidon de maïs, conseillé dans le document US 8 283 274, pour améliorer le transfert de matière. Cependant, cette porosité ne participe pas à la capacité d'adsorption et par conséquent l'amélioration du transfert de matière macroporeux se fait alors au détriment de la capacité d'adsorption volumique. Par conséquent, cette voie d'amélioration du transfert de matière macroporeux s'avère très limitée.

Pour estimer l'amélioration de la cinétique de transfert, il est possible d'utiliser la théorie des plateaux décrite par Ruthven dans « Principles of Adsorption and Adsorption Processes », ibid., pages 248-250. Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Pour une structure zéolithique donnée, une taille d'adsorbant donnée et une température de fonctionnement donnée, les diffusivités sont fixées, et un des moyens pour améliorer le transfert de matière consiste à réduire le diamètre des cristaux. Un gain sur le transfert de matière global sera ainsi obtenu en réduisant la taille des cristaux.

L'homme du métier va donc chercher à réduire le plus possible le diamètre des cristaux de zéolithes afin d'améliorer le transfert de matière.

Le brevet CN 1 267 185, revendique ainsi des adsorbants contenant 90% à 95% de zéolithe BaX ou BaKX pour la séparation du paraxylène, dans lesquels les cristaux de zéolithe X sont de taille comprise entre 0,1 $\mu$m et 0,4 $\mu$m et ceci afin d'améliorer les performances en transfert de matière. De même, la demande US 2009/0326308 décrit un procédé de séparation des isomères du xylène dont les performances ont été améliorées par l'utilisation d'adsorbants à base de cristaux de zéolithe X de taille inférieure à 0,5 $\mu$m.

La demanderesse a néanmoins observé que la synthèse, la filtration, la manipulation et l'agglomération de cristaux de zéolithe dont la taille est inférieure à 0,5 $\mu$m mettent en œuvre des procédés lourds, peu économiques et donc difficilement industrialisables.

De plus, de tels adsorbants comportant des cristaux de taille inférieure à 0,5 $\mu$m s'avèrent également plus fragiles, et il devient alors nécessaire d'augmenter le taux de liant d'agglomération afin de renforcer la cohésion des cristaux entre eux au sein de l'adsorbant. Toutefois, l'augmentation du taux de liant d'agglomération conduit à une densification des adsorbants, à l'origine d'une augmentation de la résistance diffusionnelle macroporeuse. Ainsi, malgré une résistance diffusionnelle intra-cristalline réduite du fait de la diminution de la taille des cristaux, l'augmentation de la résistance diffusionnelle macroporeuse en raison de la densification de l'adsorbant ne permet pas une amélioration du transfert global. Par ailleurs, l'augmentation du taux de liant ne permet pas d'obtenir une bonne capacité d'adsorption.

Il apparaît donc difficile d'obtenir des adsorbants avec toutes les propriétés suivantes réunies :

- un transfert de matière au sein de l'adsorbant le plus rapide possible, c'est-à-dire une résistance au transfert de matière la plus faible possible et idéalement quasi nulle,
- des propriétés de sélectivité d'adsorption du paraxylène vis-à-vis des autres molécules C8 aromatiques élevées pour assurer une séparation efficace,

- une capacité d'adsorption la plus grande possible (i.e. une teneur en zéolithe (phase cristalline active au sens de l'adsorption) la plus grande possible).
- une résistance mécanique à l'écrasement optimale.

La demanderesse a mis au point un adsorbant zéolithique présentant un compromis entre sélectivité d'adsorption du paraxylène maximale, capacité d'adsorption maximale et résistance au transfert de matières minimale, c'est-à-dire transport des molécules au sein de l'adsorbant le plus rapide possible.

La demanderesse a également mis au point un procédé de préparation desdits adsorbants, et en particulier un procédé de préparation desdits adsorbants plus économique que les procédés décrits dans l'art antérieur qui pourraient conduire auxdits adsorbants. Les adsorbants selon la présente invention s'avèrent particulièrement efficaces pour la séparation de mélanges gazeux ou liquides d'isomères, plus particulièrement des xylènes et notamment pour la séparation de paraxylène très pur à partir d'une charge d'hydrocarbures aromatiques contenant des isomères à 8 atomes de carbone.

Dans le texte qui suit, et à moins d'une autre indication, les bornes d'un domaine de valeurs sont comprises dans ce domaine, notamment dans les expressions « compris entre ... et ... » et « allant de ... à ... ».

## Adsorbant selon l'invention

Ainsi, et selon un premier objet, la présente invention concerne un adsorbant comprenant une phase zéolithique et une phase non zéolithique, ledit adsorbant présentant :

- une surface externe inférieure ou égale à 30 $m^2.g^{-1}$, de préférence inférieure ou égale à 20 $m^2.g^{-1}$,
- une phase zéolithique comprenant au moins une zéolithe de structure FAU de type X,
- et une distribution de diamètre de pores, déterminée par intrusion de mercure selon la norme ASTM D4284-83 et exprimée par la distribution en volume $dV/d\log D_{Hg}$, où $D_{Hg}$ est le diamètre apparent des pores, et V, le volume poreux, dont le mode est compris entre 100 nm et 250 nm, bornes incluses.

L'invention ne concerne pas le cas de distributions bimodales voire multimodales, c'est-à-dire de distribution où il existe plusieurs zones de concentration des valeurs séparées par des discontinuités. De telles distributions sont caractéristiques de la présence de plusieurs populations de pores de diamètres distincts.

Avantageusement, le volume microporeux de l'adsorbant selon l'invention, évalué par la méthode de t-plot à partir de l'isotherme d'adsorption d'azote ($N_2$) à une température de 77K, est supérieur à 0,200 $cm^3.g^{-1}$. Ladite isotherme d'adsorption d'azote est mesurée, après dégazage sous vide (P < 6,7.10$^{-4}$ Pa), à une température comprise entre 300°C et 450°C pendant une durée allant de 9 heures à 16 heures, de préférence à 400°C pendant 10 heures.

Avantageusement, l'adsorbant selon la présente invention comprend une phase non zéolithique dans une teneur comprise entre 2% et 8% poids par rapport au poids total de l'adsorbant. Selon un autre mode de réalisation préféré de l'invention, l'adsorbant comprend du baryum, ou du baryum et du potassium.

La surface externe de l'adsorbant zéolithique de l'invention est calculée par la méthode du t-plot à partir de l'isotherme d'adsorption d'azote à une température de 77K, après dégazage sous vide (P < 6,7.10$^{-4}$ Pa), à une température comprise entre 300°C et 450°C pendant une durée allant de 9 heures à 16 heures, de préférence à 400°C pendant 10 heures. La surface externe des cristaux de la zéolithe FAU utilisée lors de l'agglomération est mesurée de la même manière. La surface externe est une composante de la surface B.E.T qui représente la surface poreuse totale, exprimée en $m^2/g$ (S B.E.T = surface microporeuse + surface externe).

Selon un aspect préféré, la teneur en baryum (Ba) de l'adsorbant zéolithique de l'invention, exprimée en oxyde de baryum (BaO), est supérieure à 10%, de préférence supérieure à 15%, de manière très préférée supérieure à 20%, de manière encore plus préférée supérieure à 23%, voire supérieure à 33% en poids par rapport au poids total de l'adsorbant, et avantageusement, la teneur en baryum est comprise entre 23% et 42%, et typiquement entre 30% et 40%, bornes incluses, en poids par rapport au poids total de l'adsorbant.

Selon un autre aspect préféré, la teneur en potassium (K) de l'adsorbant zéolithique de l'invention, exprimée en oxyde de potassium ($K_2O$), est inférieure à 25%, de préférence comprise entre 0 et 20%, de manière encore plus préférée comprise entre 0 et 15% et de manière très préférée de 0 à 10%, bornes incluses, en poids par rapport au poids total de l'adsorbant.

Selon encore un autre mode de réalisation préféré, la teneur totale en ions alcalins ou alcalino-terreux, autres que baryum et potassium, exprimée en teneur totale d'oxydes d'ions alcalins ou alcalino-terreux autres que l'oxyde de baryum BaO et l'oxyde de potassium $K_2O$, est comprise entre 0 et 5%, bornes incluses, par rapport au poids total de l'adsorbant.

De manière avantageuse, l'adsorbant zéolithique selon l'invention présente un volume total contenu dans les macropores et les mésopores (somme du volume macroporeux et du volume mésoporeux), mesuré par intrusion de mercure selon la norme ASTM D4284-83, compris entre 0,15 cm$^3$.g$^{-1}$ et 0,5 cm$^3$.g$^{-1}$, de préférence compris entre 0,20 cm$^3$.g$^{-1}$ et 0,40 cm$^3$.g$^{-1}$ et de manière très préférée compris entre 0,20 cm$^3$.g$^{-1}$ et 0,35 cm$^3$.g$^{-1}$, toutes ces plages de valeurs s'entendant bornes incluses.

Selon un mode de réalisation préféré de la présente invention, l'adsorbant zéolithique comprend à la fois des macropores, des mésopores et des micropores. Par « macropores », on entend des pores dont le diamètre est strictement supérieur à 50 nm. Par « mésopores », on entend des pores dont le diamètre est compris entre 2 nm et 50 nm, bornes incluses. Par « micropores », on entend des pores dont le diamètre est inférieur à 2 nm.

En outre, l'adsorbant de l'invention présente avantageusement un ratio (volume macroporeux)/(volume macroporeux + volume mésoporeux) compris entre 0,2 et 1, de manière très préférée compris entre 0,6 et 1, bornes incluses.

On préfère également, dans le cadre de la présente invention, un adsorbant zéolithique dont le volume microporeux, évalué par la méthode de t-plot à partir de l'isotherme d'adsorption d'azote (N$_2$) à une température de 77 K, est supérieur à 0,200 cm$^3$.g$^{-1}$, de préférence compris entre 0,205 cm$^3$.g$^{-1}$ et 0,300 cm$^3$.g$^{-1}$ et de préférence encore compris entre 0,205 cm$^3$.g$^{-1}$ et 0,290 cm$^3$.g$^{-1}$.

Dans le cadre de la présente invention, la résistance mécanique est mesurée par la méthode Shell série SMS1471-74 adaptée pour des agglomérés de taille inférieure à 1,6 mm. Cette résistance mécanique, mesurée pour l'adsorbant zéolithique défini précédemment, est généralement comprise entre 1,5 MPa et 4 MPa, de préférence entre 1,7 MPa et 4 MPa de préférence encore entre 1,8 MPa et 4 MPa et de manière tout à fait préférée entre 2 MPa et 4 MPa, bornes incluses.

Selon encore un autre mode de réalisation préféré, l'adsorbant zéolithique selon l'invention présente un rapport atomique Si/Al compris entre 1,00 et 1,50, bornes incluses, de préférence entre 1,05 et 1,50, bornes incluses et de manière plus préférée entre 1,15 et 1,50, bornes incluses.

Parmi les zéolithes de structure FAU et de type X, il est maintenant communément admis de reconnaître entre autres deux sous-groupes dénommés zéolithes LSX et zéolithes MSX. Les zéolithes LSX présentent un rapport atomique Si/Al égal à environ 1 et les zéolithes MSX présentent un rapport atomique Si/Al compris entre environ 1,05 et environ 1,15, bornes incluses. Selon un mode de réalisation préféré, la au moins une zéolithe FAU est une zéolithe X de rapport atomique Si/Al compris entre 1,15 et 1,50, bornes incluses. Selon un autre mode de réalisation préféré, la au moins une zéolithe X est une zéolithe de type LSX de rapport atomique Si/Al est égal à environ 1.

Dans l'adsorbant zéolithique de la présente invention, et selon un mode de réalisation préféré, par « zéolithe FAU de type X », on entend les zéolithes FAU de type X définies ci-dessus, ces dites zéolithes étant à porosité hiérarchisée c'est-à-dire, les zéolithes de type X à porosité hiérarchisée (ou zéolithe XPH), les zéolithes de type MSX à porosité hiérarchisée (ou MSXPH) et les zéolithes de type LSX à porosité hiérarchisée (ou LSXPH), et plus particulièrement les zéolithes FAU à porosité hiérarchisée et de rapport atomique Si/Al compris entre 1,00 et 1,50, bornes incluses, de préférence entre 1,05 et 1,50, de préférence encore entre 1,05 et 1,40, bornes incluses, et de manière encore plus préférée, entre 1,15 et 1,40, bornes incluses.

L'invention comprend également les adsorbants zéolithiques comprenant des mélanges de deux ou plusieurs zéolithes FAU à porosité hiérarchisée telles qu'elles viennent d'être définies.

Par « zéolithe à porosité hiérarchisée », on entend une zéolithe possédant à la fois des micropores et des mésopores, autrement dit une zéolithe à la fois microporeuse et mésoporeuse. Par « zéolithe mésoporeuse », on entend une zéolithe dont les cristaux zéolithiques microporeux présentent, conjointement à la microporosité, des cavités internes de taille nanométrique (mésoporosité), facilement identifiables par observation au moyen d'un Microscope Électronique à Transmission (MET ou « TEM » en langue anglaise), comme décrit par exemple dans US 7 785 563 : l'observation par microscopie électronique à transmission (MET) permet de vérifier si les cristaux zéolithiques sont des cristaux de zéolithe pleins (i.e. non mésoporeux) ou des agrégats de cristaux de zéolithes pleins ou des cristaux mésoporeux ou des agrégats de cristaux mésoporeux.

La structure cristalline de la zéolithe FAU de type X dans l'adsorbant zéolithique de la présente invention, est identifiable par diffraction des rayons X (connue de l'homme du métier sous l'acronyme DRX).

Selon un autre mode de réalisation préféré, aucune structure zéolithique autre que la structure FAU, de préférence aucune structure zéolithique autre que la structure faujasite X, n'est détectée par diffraction des rayons X dans l'adsorbant zéolithique de la présente invention.

Par aucune structure zéolithique autre que la structure FAU, on entend moins de 5% en poids borne incluse, de préférence moins de 2% en poids borne incluse, d'une ou de plusieurs autres phases zéolithiques autres que la structure FAU. La fraction massique déterminée par DRX, technique décrite ci-après, est exprimée par rapport au poids total de l'adsorbant.

La phase non zéolithique (PNZ) comprend entre autres un liant d'agglomération utilisé dans le mode de préparation pour assurer la cohésion des cristaux entre eux, d'où le terme « aggloméré » ou « aggloméré zéolithique » employé parfois en lieu et place du terme « adsorbant zéolithique » de l'invention, tel que décrit précédemment.

Dans la présente invention, le terme « liant » signifie un liant d'agglomération qui permet d'assurer la cohésion des cristaux de zéolithe(s) dans l'adsorbant zéolithique (ou matériau zéolithique aggloméré) de l'invention. Ce liant se distingue en outre des cristaux de zéolithe(s) en ce qu'il ne présente pas de structure cristalline zéolithique après calcination, raison pour laquelle le liant est souvent qualifié d'inerte, et plus précisément inerte vis-à-vis de l'adsorption et de l'échange ionique.

Selon encore un autre mode de réalisation préféré, la fraction massique de zéolithe FAU, la zéolithe FAU étant de préférence une zéolithe de type X, est supérieure ou égale à 85%, de préférence supérieure ou égale à 90% par rapport au poids total d'adsorbant de la présente invention, le complément à 100% étant de préférence constitué de phase non zéolithique (PNZ). Selon un aspect particulièrement avantageux, la fraction massique de zéolithe FAU est comprise entre 92% et 98%, de préférence comprise entre 94% et 98% en poids, bornes incluses, par rapport au poids total d'adsorbant de la présente invention, le complément à 100% étant de préférence constitué de phase non zéolithique.

Comme déjà indiqué, la fraction massique de zéolithe(s) (taux de cristallinité) de l'adsorbant selon l'invention peut être déterminée par analyse par diffraction de rayons X, connue de l'homme du métier sous l'acronyme DRX.

Selon un mode de réalisation préféré, l'adsorbant zéolithique selon l'invention présente une perte au feu, mesurée à 950°C selon la norme NF EN 196-2, inférieure ou égale à 7,7%, de préférence comprise entre 0 et 7,7%, de manière préférée entre 3,0% et 7,7%, de manière encore préférée entre 3,5% et 6,5% et avantageusement entre 4,5% et 6,0%, bornes incluses.

Sauf indication contraire dans la présente description, les proportions indiquées sont des proportions pondérales, comptées pour les constituants solides en équivalents calcinés, sur la base de calcination réalisée à 950°C pendant 1 heure.

## Procédé de préparation de l'adsorbant selon l'invention

Un autre objet de l'invention concerne un procédé de préparation de l'adsorbant zéolithique selon l'invention, ledit procédé comprenant au moins les étapes de :

a) agglomération de cristaux d'au moins une zéolithe FAU, de préférence de type X, présentant une surface externe, mesurée par adsorption d'azote, supérieure à 20 $m^2.g^{-1}$, de préférence comprise entre 20 $m^2.g^{-1}$ et 200 $m^2.g^{-1}$, bornes incluses, de préférence encore comprise entre 40 $m^2.g^{-1}$ et 150 $m^2.g^{-1}$, bornes incluses, avec un liant comprenant de préférence au moins 80% d'argile ou d'un mélange d'argiles et avec jusqu'à 5% d'additifs ainsi qu'avec la quantité d'eau qui permet la mise en forme du matériau aggloméré, puis séchage et calcination des agglomérats ;

b) zéolithisation d'une partie du liant par mise en contact des agglomérats obtenus à l'étape a) avec une solution basique aqueuse, éventuellement en présence d'au moins un agent structurant ;

c) élimination optionnelle de l'agent structurant éventuellement présent ;

d) échange(s) cationique(s) des agglomérats de l'étape b) ou c) par mise en contact avec une solution d'ions baryum ou d'ions baryum et d'ions potassium ;

e) échange cationique éventuel supplémentaire des agglomérats de l'étape d) par mise en contact avec une solution d'ions potassium ;

f) lavage et séchage des agglomérats obtenus aux étapes d) ou e), à une température comprise entre 50°C et 150°C ; et

g) obtention de l'adsorbant zéolithique selon l'invention par activation des agglomérats obtenus à l'étape f) sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température comprise entre 100°C et 400°C, de préférence entre 200°C et 300°C.

Dans un mode de réalisation préféré du procédé de préparation de l'adsorbant zéolithique de la présente invention, le séchage des agglomérats à l'étape a) ci-dessus est généralement réalisé à une température comprise entre 50°C et 150°C, et la calcination des agglomérats séchés est généralement réalisée sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température supérieure à 150°C, typiquement comprise entre 180°C et 800°C, préférentiellement entre 200°C et 650°C, pendant quelques heures, par exemple de 2 heures à 6 heures.

Selon un mode de réalisation de la présente invention, les cristaux de zéolithe FAU utilisés lors de l'étape d'agglomération (étape a) présentent un diamètre moyen en nombre compris entre 1 $\mu$m et 20 $\mu$m, bornes incluses, de préférence encore compris entre 1,5 $\mu$m et 20 $\mu$m, bornes incluses, plus préférentiellement entre 1,8 $\mu$m et 10 $\mu$m, bornes incluses, mieux encore entre 2 $\mu$m et 10 $\mu$m, bornes incluses, et de préférence encore entre 2 $\mu$m et 8 $\mu$m, bornes incluses.

Dans le présent document, on emploie l'appellation indifféremment « diamètre moyen en nombre » ou bien « taille », en particulier pour les cristaux de zéolithe. La méthode de mesure de ces grandeurs est explicitée plus loin dans la description.

Selon un mode de réalisation préféré, la zéolithe de structure FAU est telle que définie et présente avantageusement un rapport atomique Si/Al compris de préférence entre 1,00 et 1,50, de préférence entre 1,05 et 1,50, de préférence encore entre 1,05 et 1,40, de manière plus préférée entre 1,10 et 1,40 et de manière encore plus préférée compris entre 1,15 et 1,40, bornes incluses.

Comme indiqué précédemment, la surface externe des cristaux mis en œuvre dans l'étape a) du procédé décrit ci-dessus est calculée par la méthode du t-plot à partir de l'isotherme d'adsorption d'azote à une température de 77K, après dégazage sous vide (P < 6,7.10⁻⁴ Pa), à une température comprise entre 300°C et 450°C pendant une durée allant de 9 heures à 16 heures, de préférence à 400°C pendant 10 heures.

Selon un mode de réalisation préféré, la zéolithe FAU utilisée à l'étape a) est une zéolithe FAU à porosité hiérarchisée. Les cristaux de zéolithe FAU à porosité hiérarchisée présentant une importante surface externe peuvent être obtenus selon diverses méthodes connues de l'homme du métier et par exemple selon la synthèse décrite par Inayat et coll. (Angew. Chem. Int. Ed., (2012), 51, 1962-1965).

Il est également possible de préparer lesdits cristaux par synthèse par ensemencement et/ou par ajustement des conditions opératoires de synthèse tels que le rapport SiO₂/Al₂O₃, la teneur en sodium et l'alcalinité du mélange de synthèse ou encore selon des procédés de post-traitement de cristaux de zéolithe FAU conventionnels et connus de l'homme du métier.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude (NaOH) afin d'éliminer les résidus aluminiques formés, comme décrit par exemple par D. Verboekend et coll. (Adv. Funct. Mater., 22, (2012), pp. 916-928), soit encore par des traitements qui associent l'action d'un acide et celle d'un agent structurant qui améliorent l'efficacité du traitement acide, comme décrit par exemple dans la demande WO2013/106816.

Les procédés de synthèse directe de ces zéolithes (c'est-à-dire des procédés de synthèse autre que le post-traitement) sont préférés et font généralement intervenir un ou plusieurs agents structurants ou gabarits sacrificiels.

Les gabarits sacrificiels pouvant être utilisés peuvent être de tout type connu de l'homme du métier et notamment ceux décrits dans la demande WO 2007/043731. Selon un mode de réalisation préféré, le gabarit sacrificiel est avantageusement choisi parmi les organosilanes et plus préférentiellement parmi le chlorure de [3-(triméthoxysilyl)propyl]-octadécyldiméthylammonium, le chlorure de [3-(triméthoxysilyl)propyl]hexadécyldiméthyl-ammonium, le chlorure de [3-(triméthoxysilyl)propyl]dodécyldiméthylammonium, le chlorure de [3-(triméthoxysilyl)propyl]octylammonium, la N-[3-(triméthoxysilyl)-propyl]-aniline, le 3-[2-(2-amino-éthylamino)éthylamino]propyltriméthoxysilane, la N-[3-(triméthoxysilyl)propyl]-N'-(4-vinylbenzyl)éthylènediamine, le triéthoxy-3-(2-imidazolin-1-yl)propylsilane, la 1-[3-(triméthoxysilyl)propyl]urée, la N-[3-(triméthoxysilyl)propyl]-éthylènediamine, le [3-(diéthylamino)propyl]triméthoxysilane, le (3-glycidyloxypropyl)triméthoxysilane, le méthacrylate de 3-(triméthoxysilyl)propyle, le [2-(cyclohexényl)éthyl]triéthoxysilane, le dodécyltriéthoxysilane, l'hexadécyltriméthoxysilane, le (3-aminopropyl)triméthoxysilane, le (3-mercaptopropyl)triméthoxysilane, le (3-chloropropyl)triméthoxysilane, ainsi que les mélanges de deux ou plusieurs d'entre eux en toutes proportions.

Parmi les gabarits sacrificiels listés ci-dessus, on préfère tout particulièrement le chlorure de [3-(triméthoxysilyl)propyl]octadécyldiméthylammonium, ou TPOAC.

On peut également utiliser des gabarits sacrificiels de masse molaire plus élevée et par exemple les PPDA (Polymer Poly-DiallyldimethylAmmonium), PVB (PolyVinyl Butyral) et autres composés oligomères connus dans le domaine pour augmenter le diamètre des mésopores.

Selon un mode de réalisation préféré du procédé de la présente invention, on procède, à l'étape a), à l'agglomération de cristaux d'au moins une zéolithe FAU à porosité hiérarchisée, comme décrit précédemment, préparée en présence d'un gabarit sacrificiel destiné à être éliminé.

Cette élimination peut être réalisée selon les méthodes connues de l'homme du métier, par exemple par calcination, et de manière non limitative, la calcination des cristaux de zéolithe comprenant le gabarit sacrificiel peut être effectuée sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une ou des températures supérieures à 150°C, typiquement comprises entre 180°C et 800°C, préférentiellement entre 200°C et 650°C, pendant quelques heures, par exemple entre 2 et 6 heures. La nature des gaz, les rampes de montée en température et les paliers successifs de températures, leurs durées seront adaptées en fonction de la nature du gabarit sacrificiel.

L'étape supplémentaire d'élimination de l'éventuel gabarit sacrificiel peut être effectuée à tout moment au cours du procédé de préparation de l'adsorbant zéolithique de l'invention. L'élimination dudit gabarit sacrificiel peut ainsi avantageusement être effectuée par calcination des cristaux de zéolithe avant l'étape d'agglomération a), ou encore de manière concomitante avec la calcination de l'adsorbant lors de l'étape a).

On ne sortirait toutefois pas du cadre de l'invention si l'agglomération de l'étape a) comprenait l'agglomération de plusieurs zéolithes FAU à porosité hiérarchisée obtenues selon des modes différents.

La synthèse de zéolithe de type FAU se fait généralement en milieu sodique (hydroxyde de sodium et donc cation Na⁺). Les cristaux de zéolithe FAU ainsi obtenus comprennent majoritairement, voire exclusivement des cations sodium. On ne sortirait toutefois pas du cadre de l'invention en utilisant des cristaux ayant subi un ou plusieurs échanges cationiques, entre la synthèse sous forme Na, avant ou après l'élimination éventuelle du gabarit sacrificiel si cette étape est opérée avant la mise en œuvre à l'étape a). Dans ce cas, l'étape d) et éventuellement l'étape e) d'échange devien(nen)t par conséquent éventuellement non nécessaire(s).

La taille des cristaux de zéolithe FAU utilisés à l'étape a) et des cristaux de zéolithe FAU dans les adsorbants selon l'invention est mesurée par observation au microscope électronique à balayage (MEB).

L'agglomération et la mise en forme (étape a) peuvent être réalisées selon toutes les techniques connues de l'homme de l'art, et en particulier selon une ou plusieurs des techniques choisies parmi extrusion, compactage, agglomération sur assiette granulatrice, tambour granulateur, atomisation et autres.

Les proportions de liant d'agglomération (voir définition plus loin) et de zéolithe mises en œuvre sont de 8 parties à 15 parties en poids de liant pour 92 parties à 85 parties en poids de zéolithe.

À l'issue de l'étape a), les adsorbants agglomérés les plus fins peuvent être éliminés par cyclonage et/ou tamisage et/ou les agglomérés trop gros par tamisage ou concassage, dans le cas d'extrudés, par exemple. Les adsorbants ainsi obtenus qu'ils soient sous forme de billes, d'extrudés ou autres, ont de préférence un diamètre moyen en volume, ou leur longueur (plus grande dimension lorsqu'ils ne sont pas sphériques), comprise entre 0,2 mm et 2 mm, et en particulier comprise entre 0,2 mm et 0,8 mm et de préférence entre 0,40 mm et 0,65 mm, bornes incluses.

Le liant utilisable dans le cadre de la présente invention peut donc être choisi parmi les liants classiques connus de l'homme du métier, et de préférence choisis parmi les argiles et les mélanges d'argiles.

Les argiles sont de préférence choisies parmi : kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

De préférence, le liant d'agglomération mis en œuvre à l'étape a) contient au moins une argile zéolithisable, de préférence choisie dans la famille des kaolins, kaolinites, nacrites, dickites, halloysites et métakaolins, et leurs mélanges. Le kaolin est préféré et le plus couramment utilisé. Le liant d'agglomération mis en œuvre à l'étape a) peut également contenir d'autres liants minéraux tels que bentonite, attapulgite, et autres. Par argile zéolithisable, on entend une argile ou un mélange d'argiles qui sont susceptibles d'être converties en matière zéolithique, le plus souvent par action d'une solution basique aqueuse.

Parmi les additifs éventuellement mis en œuvre à l'étape a), on peut trouver une source de silice de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Lors de l'étape a), outre le ou les cristaux de zéolithe(s), le liant peut comprendre également un ou plusieurs additifs. Les additifs sont préférentiellement organiques, par exemple de la lignine, de l'amidon, de la carboxyméthylcellulose, des molécules tensioactives (cationiques, anioniques, non ioniques ou amphotères), destinées à faciliter la manipulation de la pâte zéolithe(s)/argile(s) par modification de la rhéologie et/ou du pouvoir collant ou à conférer aux adsorbants finaux des propriétés satisfaisantes, notamment de macroporosité.

On peut citer de manière préférentielle mais non exhaustive, les méthyl-celluloses et leurs dérivés, les lignosulfonates, les acides polycarboxyliques et les acides de copolymères carboxyliques, leurs dérivés aminés et leurs sels, notamment les sels alcalins et les sels d'ammonium. Les additifs sont introduits à raison de 0 à 5%, de préférence de 0,1% à 2%, en poids par rapport au poids total de l'adsorbant.

L'observation MEB de l'adsorbant zéolitique permet de confirmer la présence de phase non zéolithique comprenant par exemple du liant d'agglomération ou toute autre phase amorphe dans les adsorbants.

Pour la calcination comprise dans l'étape a), la nature des gaz, les rampes de montée en température et les paliers successifs de températures, ainsi que leurs durées respectives, seront adaptés notamment en fonction de la nature du gabarit sacrificiel à éliminer et en fonction de la nature du liant mis en œuvre à l'étape d'agglomération a).

De même, l'étape b) de zéolithisation est une étape maintenant bien connue de l'homme du métier qui peut être conduite selon toute méthode décrite dans l'art antérieur, la solution aqueuse alcaline utilisée pouvant être une solution aqueuse d'hydroxyde de sodium ou de potassium, l'utilisation d'hydroxyde de sodium étant tout particulièrement préférée. En règle générale, la concentration de la solution alcaline alcaline de zéolithisation est comprise entre 0,5 M et 5 M.

La zéolithisation s'opère de préférence à chaud, à une température supérieure à la température ambiante, par exemple comprise entre la température ambiante (environ 20°C) et la température d'ébullition de la solution alcaline de zéolithisation, et par exemple à des températures de l'ordre de 80°C à 100°C. La durée du processus de zéolithisation est généralement comprise entre quelques dizaines de minutes et quelques heures, habituellement entre environ 1 heure et 8 heures.

Selon un mode de réalisation du procédé de la présente invention, l'étape b) de zéolithisation d'une partie du liant d'agglomération peut être réalisée en présence d'au moins un agent structurant ou gabarit sacrificiel destiné à être éliminé selon les méthodes connues de l'homme du métier, par exemple par calcination, la présence de l'agent structurant ayant pour but de créer une certaine mésoporosité dans l'aggloméré de l'invention ainsi obtenir un aggloméré zéolithique mésoporeux.

La nature de l'agent structurant ou gabarit sacrificiel peut-être de tout type connu de l'homme du métier et en particulier choisi parmi ceux décrits plus haut pour la synthèse de zéolithe à porosité hiérarchisée.

La quantité d'agent structurant, lorsque celui-ci est présent lors de l'étape de zéolithisation, peut varier dans de grandes proportions selon le degré de mésoporosité recherché, et est avantageusement comprise entre 0,1% et 50%, de préférence entre 0,1% et 33%, de préférence encore entre 1% et 30%, avantageusement entre 5% et 30%, en poids par rapport au poids d'argile(s).

L'étape c) d'élimination éventuelle de l'agent structurant éventuellement introduit lors de l'étape de zéolithisation b) et visant à convertir une partie du liant d'agglomération en zéolithe mésoporeuse, peut être réalisée par tout moyen connu de l'homme du métier et en particulier par traitement thermique, généralement à une température supérieure à 150°C, typiquement comprise entre 180°C et 650°C, préférentiellement entre 200°C et 600°C. Dans ce cas, l'étape g) d'activation effectuée à haute température permet également l'élimination de l'agent structurant rendant ainsi avantageusement possible de ne pas réaliser l'étape e) d'élimination dudit agent structurant qui sera de fait éliminé lors de l'activation à l'étape g).

Les étapes d'échange(s) cationique(s) d) et e) décrites plus haut s'effectuent selon les méthodes classiques connues de l'homme du métier, et le plus souvent par mise en contact des adsorbants issus de l'étape a) avec un sel de baryum, tel que le chlorure de baryum ($BaCl_2$) et/ou de potassium (KCl) et/ou de baryum et de potassium, en solution aqueuse à une température comprise entre la température ambiante et 100°C, et de préférence comprise entre 80°C et 100°C afin d'obtenir rapidement des teneurs élevées en baryum, i.e. des teneurs de préférence supérieures à 10%, de préférence supérieures à 15%, de manière très préférée supérieures à 20%, de manière encore plus préférée supérieures à 23%, voire supérieures à 33%, exprimées en poids d'oxyde de baryum par rapport à la masse totale de l'adsorbant.

Avantageusement, la teneur en baryum exprimée en oxyde de baryum est comprise entre 23% et 42%, et typiquement entre 30% et 40%, bornes incluses, en poids par rapport au poids total de l'adsorbant. On préfère opérer avec un large excès d'ions baryum par rapport aux cations de la zéolithe que l'on souhaite échanger, typiquement un excès de l'ordre de 10 à 12, avantageusement en procédant par échanges successifs.

Un échange éventuel au potassium à l'étape e) peut être pratiqué avant et/ou après l'échange au baryum (étape b). Il est également possible d'agglomérer à l'étape a) des cristaux de zéolithe FAU contenant déjà des ions baryum ou potassium ou baryum et potassium (pré-échange des cations présents dans la zéolithe de type FAU de départ, typiquement cations sodium, par des ions baryum ou potassium ou baryum et potassium avant l'étape a) et s'affranchir (ou non) des étapes d) et/ou e).

De manière surprenante, la demanderesse a observé que l'étape d'échange cationique, qui peut être délicate en raison de la relative fragilité de la structure des cristaux de zéolite à porosité hiérarchisée n'affecte pas les propriétés intrinsèques de surface externe et du volume microporeux (ramené à la masse de l'adsorbant une fois échangé) desdits cristaux de zéolithe à porosité hiérarchisée.

Après la ou les étape(s) d'échange(s) cationique(s), on procède ensuite à un lavage, généralement et de préférence à l'eau, puis à un séchage de l'adsorbant ainsi obtenu. L'activation qui suit le séchage, est conduite de manière classique, selon les méthodes connues de l'homme du métier, par exemple à une température en général comprise entre 100°C et 400°C, de préférence entre 200°C et 300°C. L'activation est opérée pendant une durée déterminée en fonction de la perte au feu souhaitée. Cette durée est généralement comprise entre quelques minutes et quelques heures, typiquement de 1 à 6 heures.

### Utilisation des adsorbants selon l'invention

La présente invention concerne également les utilisations des adsorbants zéolithiques décrits ci-dessus comme agents d'adsorption susceptibles de remplacer avantageusement les agents d'adsorption décrits dans la littérature, à base de cristaux conventionnels de zéolithe de type FAU, et notamment dans les utilisations listées ci-dessous :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- séparation des alcools polyhydriques, tels que les sucres.

Selon un autre objet, la présente invention concerne un procédé de séparation des isomères de xylènes en phase gaz ou en phase liquide mettant en œuvre au moins un adsorbant zéolithique tel que défini précédemment.

L'invention concerne notamment un procédé de séparation de paraxylène à partir d'une charge à traiter de coupes d'isomères aromatiques à 8 atomes de carbone, utilisant, comme agent d'adsorption du paraxylène, un adsorbant zéolithique tel que défini précédemment.

On peut ainsi séparer le produit désiré (paraxylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Les conditions opératoires d'une unité industrielle d'adsorption en lit mobile simulé fonctionnant à contre-courant sont en général les suivantes :

- nombre de lits : 4 à 24,
- nombre de zones : au moins 4 zones de fonctionnement, chacune étant localisée entre un point d'alimentation (flux de charge à traiter ou flux de désorbant) et un point de soutirage (flux de raffinat ou flux d'extrait);
- température comprise entre 100°C et 250°C, de préférence entre 140°C et 190°C,
- pression de l'unité industrielle comprise entre la pression de bulle des xylènes (ou du toluène lorsque le toluène est choisi comme désorbant) à la température du procédé et 3 MPa,
- rapport des débits désorbant/charge compris entre 0,7 et 2,5, de préférence entre 0,7 et 2,0, par exemple entre 0,9 et 1,8 pour une unité d'adsorption seule (dite "stand alone") et entre 0,7 et 1,4 pour une unité d'adsorption combinée à une unité de cristallisation,
- taux de recyclage compris entre 2 et 12 de préférence entre 2,5 et 6,
- temps de cycle, correspondant au temps entre deux injections de désorbant sur un lit donné : de manière avantageuse, compris entre 4 et 25 min.

On pourra sur ce sujet se référer à l'enseignement des brevets US 2 985 589, US 5 284 992 et US 5 629 467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US 4 402 832 et US 4 498 991.

Le désorbant est un solvant de désorption dont le point d'ébullition est inférieur à celui de la charge, tel que le toluène ou supérieur à celui de la charge, tel que le *para*-diéthylbenzène (PDEB). Avantageusement, le désorbant est le toluène ou le *para*-diéthylbenzène.

La sélectivité des adsorbants selon l'invention pour l'adsorption du paraxylène contenu dans des coupes aromatiques en C8 est optimale lorsque leur perte au feu mesurée à 950°C est de préférence inférieure ou égale à 7,7%, de préférence comprise entre 0 et 7,7%, de manière très préférée entre 3,0% et 7,7%, de manière plus préférée entre 3,5% et 6,5% et de manière encore plus préférée entre 4,5% et 6%, bornes incluses.

La teneur en eau dans les flux entrants constitués par les flux de charge et/ou de désorbant est préférentiellement ajustée entre 20 ppm et 150 ppm, par exemple en ajoutant de l'eau dans les flux de charge et/ou de désorbant.

Les adsorbants zéolithiques de l'invention présentent des propriétés de transfert de matière améliorées tout en maintenant des propriétés optimales de sélectivité du paraxylène, une capacité d'adsorption maximale, et en conservant une résistance mécanique élevée pour une utilisation dans un procédé de séparation du paraxylène en phase liquide, de préférence de type contre-courant simulé.

Il a été tout particulièrement observé que les adsorbants zéolithiques selon la présent invention présentent un indice combiné IC supérieur à 14, et en général supérieur à 15, ce qui est tout à fait remarquable par rapport aux valeurs d'IC observés avec les adsorbants de l'art antérieur.

L'indice IC est défini par la formule suivante :

$$IC = \frac{\text{sélectivité x capacité}}{\text{HEPT}}$$

dans laquelle :

- le paramètre « sélectivité » représente la sélectivité entre le paraxylène et le métaxylène,
- le paramètre « capacité » représente la capacité d'adsorption des xylènes exprimé en % ($cm^3$ de $C_8$-aromatiques adsorbés par $cm^3$ de colonne)
- le paramètre « HEPT » représente la hauteur équivalente de plateaux théoriques mesuré sur le paraxylène rapporté à la longueur de colonne (exprimé en %).

Les différents paramètres sont déterminés par la technique dite de perçage en phase liquide dans un test réalisé dans les conditions suivantes :

- la température d'adsorption est de 175°C,
- la vitesse superficielle (débit/section de la colonne) de circulation du liquide à la température du test est de 1,3 cm.s-1,

- le solvant de désorption utilisé est le para-diéthylbenzène,
- la composition de la charge est la suivante :

  • Paraxylène : 45% poids
  • Métaxylène : 45% poids
  • Iso-octane: 10% poids (celui-ci est utilisé comme traceur pour l'estimation des volumes non-sélectifs et n'intervient pas dans la séparation) ;

Selon encore une autre aspect, la présente invention concerne l'utilisation d'au moins un adsorbant zéolithique présentant un indice combiné IC supérieur à 14, et de préférence supérieur à 15, dans les procédés de séparation des isomères de xylènes en phase gaz ou en phase liquide, tels que ceux définis précédemment.

Dans cette utilisation ledit au moins un adsorbant zéolithique présente une résistance mécanique avantageusement supérieure à 1,5 MPa, de préférence comprise entre 1,7 MPa et 4 MPa de préférence encore entre 1,8 MPa et 4 MPa et de manière tout à fait préférée entre 2 MPa et 4 MPa, bornes incluses. Dans le cadre de la présente invention, la résistance mécanique est mesurée par la méthode Shell série SMS1471-74 adaptée pour des agglomérés de taille inférieure à 1,6 mm.

## TECHNIQUES DE CARACTÉRISATION

### Granulométrie des cristaux zéolithiques

L'estimation du diamètre moyen en nombre des cristaux de zéolithe FAU utilisés lors de l'étape a) d'agglomération et des cristaux contenus dans les adsorbants zéolithiques selon l'invention est réalisée par observation au microscope électronique à balayage (MEB).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié. La précision est de l'ordre de 3%.

### Analyse chimique des adsorbants zéolithiques - rapport Si/Al et taux d'échange :

Une analyse chimique élémentaire du produit final obtenu à l'issue des étapes a) à e) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/Al de la zéolithe utilisée lors de la préparation de l'adsorbant, ainsi que le rapport atomique Si/Al de l'adsorbant et de vérifier la qualité de l'échange ionique décrit à l'étape c) et à l'étape optionnelle d). Dans la description de la présente invention, l'incertitude de mesure du rapport atomique Si/Al est de $\pm$ 5%.

La qualité de l'échange ionique est liée au nombre de mole d'oxyde de sodium, $Na_2O$, restant dans l'adsorbant zéolithique après échange. Plus précisément, le taux d'échange par les ions baryum est estimé en évaluant le rapport entre le nombre de moles d'oxyde de baryum, BaO, et le nombre de moles de l'ensemble ($BaO + Na_2O + K_2O$). De même, le taux d'échange par les ions potassium est estimé en évaluant le rapport entre le nombre de moles d'oxyde de potassium $K_2O$ et le nombre de moles de l'ensemble ($BaO + K_2O + Na_2O$). Il est à noter que les teneurs en différents oxydes sont données, en pourcentage en poids par rapport au poids total de l'adsorbant zéolithique anhydre.

### Granulométrie des adsorbants zéolithiques :

La détermination du diamètre moyen en volume des adsorbants zéolithiques obtenus à l'issus de l'étape a) d'agglomération et de mise en forme est effectuée par analyse de la distribution granulométrique d'un échantillon d'adsorbant par imagerie selon la norme ISO 13322-2:2006, en utilisant un tapis roulant permettant à l'échantillon de passer devant l'objectif de la caméra.

Le diamètre moyen en volume est ensuite calculé à partir de la distribution granulométrique en appliquant la norme ISO 9276-2:2001. Dans le présent document, on emploie l'appellation « diamètre moyen en volume » ou bien « taille » pour les adsorbants zéolithiques. La précision est de l'ordre de 0,01 mm pour la gamme de taille d'adsorbants de l'invention.

**Résistance mécanique des adsorbants zéolithiques :**

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm, mais doit être adaptée selon la granulométrie des adsorbants que l'on cherche à caractériser.

Les adsorbants de la présente invention, généralement sous forme de billes ou d'extrudés, ont en général un diamètre moyen en volume ou une longueur, i.e. plus grande dimension dans le cas des adsorbants non sphériques, comprise entre 0,2 mm et 2 mm, et en particulier comprise entre 0,2 mm et 0,8 mm et de préférence entre 0,40 mm et 0,65 mm, bornes incluses. Par conséquent, un tamis de 100 $\mu$m est utilisé à la place du tamis de 425 $\mu$m mentionné dans la méthode Shell standard SMS1471-74.

Le protocole de mesure est le suivant: un échantillon de 20 cm$^3$ d'adsorbants agglomérés, préalablement tamisé avec le tamis adapté (100 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les adsorbants agglomérés (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 $\mu$m) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5% massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

**Phase non zéolithique des adsorbants zéolithiques :**

La teneur en phase non zéolithique PNZ, par exemple le taux de liant d'agglomération et de toute autre phase amorphe est calculé selon l'équation suivante :

$$PNZ = 100 - \Sigma\ (PZ),$$

où PZ représente la somme des quantités des fractions zéolithiques X au sens de l'invention.

**Quantité massique des fractions zéolithiques des adsorbants zéolithiques**

L'identification des fractions zéolithiques contenues dans l'adsorbant est effectuée par analyse par diffraction de rayons X (DRX). Cette analyse est réalisée sur un appareil de la marque Bruker. L'identification des phases cristallines présentes dans l'adsorbant zéolithique est réalisée par comparaison avec les fiches de la base de données ICDD. Par exemple la présence de la zéolithe de type X échangée au baryum sera confirmée par comparaison des raies du diffractogramme obtenu avec la fiche ICDD n°38-0234 (« Zeolite X, (Ba) »). La quantité massique des fractions zéolithiques est évaluée à partir des intensités de pic des diffractogrammes en prenant comme référence les intensités de pic d'une référence appropriée (zéolithe de même nature chimique supposée 100% cristalline dans des conditions de traitements cationiques identiques à celles l'adsorbant considéré). Les pics, permettant de remonter à la cristallinité, sont les pics les plus intenses de la zone angulaire 2θ comprise entre 9° et 37°, à savoir les pics observés dans les plages angulaires 2θ comprises respectivement entre 11° et 13°, entre 22° et 26° et entre 31° et 33°.

**Volume microporeux, surface externe**

La cristallinité des cristaux et des adsorbants zéolithiques de l'invention est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'échantillon zéolithique (que ce soit adsorbant ou cristaux) est dégazé entre 300°C et 450°C pendant une durée comprise entre 9 heures et 16 heures, sous vide (P < 6,7.10$^{-4}$ Pa). La mesure de l'isotherme d'adsorption d'azote à 77K est ensuite effectuée sur un appareil de type ASAP 2020 M de Micromeritics, en prenant au moins 35 points de mesure à des pressions relatives de rapport $P/P_0$ compris entre 0,002 et 1.

Le volume microporeux ainsi que la surface externe sont déterminés à partir de l'isotherme obtenue, par la méthode du t-plot en appliquant la norme ISO 15901-3:2007 et en calculant l'épaisseur statistique t par l'équation de Harkins-Jura. Le volume microporeux et la surface externe sont obtenus par régression linéaire sur les points du t-plot compris entre 0,45 nm et 0,57 nm, respectivement à partir de l'ordonnée à l'origine et de la pente de la régression linéaire. Le volume microporeux évalué s'exprime en cm$^3$ d'adsorbat liquide par gramme d'adsorbant anhydre. La surface externe s'exprime en m$^2$ par gramme d'adsorbant anhydre.

**Volume macroporeux et mésoporeux, distribution de diamètre de pores par intrusion de mercure, et densité de grain**

Les volumes macroporeux et mésoporeux ainsi que la densité de grain sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (matériau granulaire zéolithique à mesurer) (de perte au feu connue) préalablement pesé, dans une cellule du porosimètre, puis, après un dégazage préalable (pression d'évacuation de 30 μm Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon. L'incrément de volume poreux dV dans lequel le mercure pénètre à chaque palier de pression est enregistré. Le nombre de palier de pression est typiquement d'environ 15 entre 0,0036 MPa et 0,2 MPa, et d'environ 90 entre 0,2 MPa et 400 MPa.

La relation entre la pression appliquée et le diamètre apparent des pores $D_{Hg}$ est établie en supposant des pores cylindriques, un angle de contact entre le mercure et la paroi des pores de 140° et une tension de surface du mercure de 485 dynes/cm. La quantité cumulée de mercure introduite en fonction de la pression appliquée est enregistrée. On fixe à 0,2 MPa la valeur à partir de laquelle le mercure remplit tous les vides inter-granulaires, et on considère qu'au-delà le mercure pénètre dans les pores du matériau granulaire. Le volume de grain (Vg) est alors calculé en soustrayant le volume cumulé de mercure à cette pression (0,2 MPa) au volume de la cellule du porosimètre, et en divisant cette différence par la masse du matériau granulaire équivalent anhydre, c'est-à-dire la masse dudit matériau corrigée de la perte au feu.

La densité de grain est l'inverse du volume de grain (Vg), et s'exprime en gramme d'adsorbant anhydre par cm$^3$.

Le volume macroporeux du matériau granulaire est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux du matériau granulaire est défini comme étant le volume cumulé de mercure introduit à une pression comprise entre 30 MPa et 400 MPa.

Dans le présent document, les volumes macroporeux et mésoporeux des adsorbants zéolithiques, exprimés en cm$^3$.g$^{-1}$, sont ainsi mesurés par intrusion de mercure et rapportés à la masse de l'échantillon en équivalent anhydre, c'est-à-dire la masse dudit matériau corrigée de la perte au feu.

La distribution de diamètre de pores déterminée par intrusion de mercure est exprimée par la distribution en volume $dV/d\log D_{Hg}$ représentée en fonction du diamètre de pores apparent des pores $D_{Hg}$. Les valeurs $dV/d\log D_{Hg}$ sont calculées à partir de l'incrément de volume poreux dV dans lequel le mercure pénètre à chaque palier de pression rapporté à la différence de diamètre de pores apparent correspondant.

**Perte au feu des adsorbants zéolithiques :**

La perte au feu est déterminée en atmosphère oxydante, par calcination de l'échantillon à l'air à une température de 950°C ± 25°C, comme décrit dans la norme NF EN 196-2 (avril 2006). L'écart type de mesure est inférieur à 0,1%.

**Caractérisation de l'adsorption en phase liquide par perçage :**

La technique utilisée pour caractériser l'adsorption de molécules en phase liquide sur un solide poreux est la technique dite de perçage, décrite par Ruthven dans « Principles of Adsorption and Adsorption Processes » (Chapitres 8 et 9, John Wiley & Sons, 1984) qui définit la technique des courbes de perçage (« breakthrough curves ») comme l'étude de la réponse à l'injection d'un échelon de constituants adsorbables. L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables.

L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

## EXEMPLES :

### Exemple A : Synthèse de zéolithe FAU à porosité hiérarchisée

La zéolithe FAU à haute surface externe est synthétisée de manière directe d'après l'article Inayat et coll. (Angew. Chem. Int. Ed., (2012), 51, 1962-1965).

### Étape 1) : Préparation du gel de croissance dans réacteur agité avec vis d'Archimède à 300 tr.min$^{-1}$.

Dans un réacteur en inox muni d'une double enveloppe chauffante, d'une sonde température et d'un agitateur, on prépare un gel de croissance en mélangeant une solution d'aluminate contenant 119 g d'hydroxyde de sodium (NaOH) à, 128 g d'alumine trihydratée ($Al_2O_3$, $3H_2O$, contenant 65,2% en poids d'$Al_2O_3$) et 195,5 g eau à 25°C en 25 minutes avec une vitesse d'agitation de 300 tr.min$^{-1}$ dans une solution de silicate contenant 565,3 g de silicate de sodium, 55,3 g de NaOH et 1997,5 g d'eau à 25°C.

La stœchiométrie du gel de croissance est la suivante : 3,48 $Na_2O$ / $Al_2O_3$ / 3,07 $SiO_2$ / 180 $H_2O$. L'homogénéisation du gel de croissance est réalisée sous agitation à 300 tr.min$^{-1}$, pendant 25 minutes à 25°C.

### Étape 2) : Introduction dans le milieu réactionnel de l'agent structurant

On introduit dans le milieu réactionnel 27,3 g de solution de TPOAC à 60% dans le MeOH avec une vitesse d'agitation de 300 tr.min$^{-1}$ (ratio molaire TPOAC/$Al_2O_3$ = 0,04). Après 5 minutes d'homogénéisation, on baisse la vitesse d'agitation à 50 tr.min$^{-1}$.

### Étape 3) : Phase de mûrissement

On maintient le milieu réactionnel agité à 50 tr.min$^{-1}$ à 25°C, pendant 22 heures, puis on démarre la cristallisation.

### Étape 4) : Cristallisation

On maintient la vitesse d'agitation à 50 tr.min$^{-1}$, et on fixe la consigne de la double enveloppe du réacteur à 80°C afin que le milieu réactionnel monte en température à 75°C en 80 minutes. Après 72 heures de palier à 75°C, on refroidit le milieu réactionnel en faisant circuler de l'eau froide dans la double enveloppe pour stopper la cristallisation.

### Étape 5) : Filtration / lavage

Les solides sont récupérés sur fritté puis lavés avec de l'eau permutée jusqu'à pH neutre.

### Étape 6) : Séchage / Calcination

Afin de caractériser le produit, le séchage est réalisé en étuve à 90°C pendant 8 heures, la perte au feu du produit séché est de 22% en poids.

La calcination du produit séché nécessaire pour libérer à la fois la microporosité (eau) et la mésoporosité en éliminant l'agent structurant est effectuée avec le profil de température suivant : 30 minutes de montée à 200°C, puis 1 heure de palier à 200°C, puis 3 heures de montée à 550°C, et enfin 1,5 heures de palier à 550°C.

Les cristaux obtenus sont identifiés par diffraction des rayons X (analyse DRX) comme étant des cristaux de faujasite. L'analyse chimique du solide donne un rapport atomique Si/Al = 1,24. Le diamètre moyen en nombre des cristaux de la zéolithe mésoporeuse (ou à porosité hiérarchisée) ainsi obtenue est de 4,5 $\mu$m

Le volume microporeux et la surface externe mesurés selon la méthode du t-plot à partir de l'isotherme d'adsorption d'azote à 77K après dégazage sous vide à 400°C pendant 10 heures sont respectivement 0,260 cm$^3$.g$^{-1}$ et 90 m$^2$.g$^{-1}$ exprimés par gramme d'adsorbant sec.

**Exemple B : Synthèse de cristaux de zéolithe X non mésoporeuse, de rapport atomique Si/Al = 1,25, de diamètre moyen en nombre de 1,0 $\mu$m et de rapport atomique Na/Al = 1**

On prépare un gel de composition molaire 3,5 Na$_2$O - 2,8 SiO$_2$ - Al$_2$O$_3$ - 130 H$_2$O, par mélange des réactifs suivants : silicate de sodium, aluminate de sodium et eau. Le gel est mûri à 35°C pendant 20 heures, et on opère une cristallisation pendant 4 heures à 100°C.

Les cristaux obtenus après filtration et lavage sont identifiés par diffraction des rayons X (analyse DRX) comme étant des cristaux de faujasite. L'analyse chimique du solide donne un rapport atomique Si/Al = 1,25. Le diamètre moyen en nombre des cristaux de zéolithe est de 1,0 $\mu$m. Le volume microporeux et la surface externe mesurés selon la méthode du t-plot à partir de l'isotherme d'adsorption d'azote à 77K après dégazage sous vide à 400°C pendant 10 heures sont respectivement 0,345 cm$^3$.g$^{-1}$ et 2 m$^2$.g$^{-1}$. exprimés par gramme d'adsorbant sec.

**Préparation des adsorbants zéolithiques**

On prépare un mélange homogène et on agglomère 1600 g de cristaux de zéolithe NaX préparée selon les modes opératoires décrit dans les exemples A ou B avec 350 g de kaolin (exprimés en équivalent calciné) et 130 g de silice colloïdale vendue sous la dénomination commerciale Klebosol® 30 (contenant 30% en poids de SiO$_2$ et 0,5% de Na$_2$O) avec la quantité d'eau qui permet l'extrusion du mélange. La perte au feu de la pâte avant extrusion est de 44%. On forme des extrudés de 1,6 mm de diamètre. Les extrudés sont séchés une nuit en étuve ventilée à 80°C. Ils sont ensuite calcinés pendant 2 heures à 550°C sous balayage à l'azote, puis 2 heures à 550°C sous balayage à l'air sec décarbonaté puis concassés de manière à récupérer des grains dont le diamètre équivalent est égal à 0,4 mm.

**Exemple 1 :** (comparatif)

*Préparation d'un adsorbant zéolithique sous forme de concassés avec une zéolithe de type X, taille des cristaux de zéolithe de 1, 0 $\mu$m et un liant de type kaolin non zéolithisé*

Des granulés (200 g) obtenus à partir de la poudre synthétisée dans l'exemple B sont échangés au moyen d'une solution de chlorure de baryum 0,5 M à 95°C en 4 étapes. À chaque étape, le ratio volume de solution sur masse de solide est de 20 mL.g$^{-1}$ et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 heures sous courant d'azote.

Le taux d'échange en baryum est de 97% et la perte au feu est de 5,4%. Le volume microporeux et la surface externe mesurés selon la méthode du t-plot à partir de l'isotherme d'adsorption d'azote à 77 K après dégazage sous vide à 400°C pendant 10 heures sont respectivement 0,226 cm$^3$.g$^{-1}$ et 16,7 m$^2$.g$^{-1}$.

Le volume total des macropores et des mésopores mesuré par porosimétrie mercure est de 0,32 cm$^3$.g$^{-1}$. La fraction de volume des macropores sur le volume total des macropores et mésopores est égale à 0,87.

La distribution de diamètre de pores est déterminée à partir de l'analyse par intrusion de mercure réalisée sur l'adsorbant et représentée par la distribution en volume $d$V/$d$logD$_{Hg}$, en fonction du diamètre apparent des pores D$_{Hg}$. La distribution présente un pic distinct dans le domaine des macropores correspondant à une distribution unimodale autour d'un mode égal à environ 350 nm.

On mesure également la résistance mécanique selon la méthode présentée dans la description de l'invention. La pression nécessaire pour obtenir 0,5% de fines est de 2,2 MPa.

**Exemple 2 :** (comparatif)

*Préparation d'un adsorbant zéolithique sous forme de concassés avec une zéolithe de type X, taille des cristaux de zéolithe de 1,0$\mu$m et un liant de type kaolin zéolithisé*

Des granulés (200 g) obtenus à partir de la poudre synthétisée dans l'exemple B sont placés dans un réacteur en verre muni d'une double enveloppe régulée à une température de 100°C $\pm$ 1°C, puis on ajoute 1,5 L d'une solution aqueuse d'hydroxyde de sodium de concentration 1 M et on laisse le milieu réactionnel sous agitation pendant une durée de 3 heures.

On procède ensuite au lavage des agglomérés en 3 opérations successives de lavage à l'eau suivi de la vidange du réacteur. On s'assure de l'efficacité du lavage en mesurant le pH final des eaux de lavage compris entre 10,0 et 10,5.

Ces agglomérés sont échangés au moyen d'une solution de chlorure de baryum 0,5 M à 95°C en 4 étapes. À chaque étape, le ratio volume de solution sur masse de solide est de 20 mL.g$^{-1}$ et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 heures sous courant d'azote.

Le taux d'échange en baryum est de 97% et la perte au feu est de 5,3%. Le volume microporeux et la surface externe mesurés selon la méthode du t-plot à partir de l'isotherme d'adsorption d'azote à 77 K après dégazage sous vide à 400°C pendant 10 heures sont respectivement 0,249 cm$^3$.g$^{-1}$ et 5 m$^2$.g$^{-1}$.

Le volume total des macropores et des mésopores mesuré par porosimétrie mercure est de 0,29 cm$^3$.g$^{-1}$. La fraction de volume des macropores sur le volume total des macropores et mésopores est égale à 0,97.

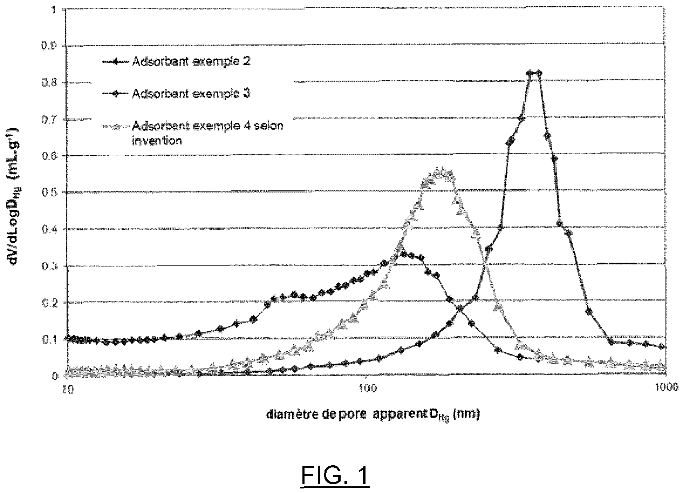

La distribution de diamètre de pores est déterminée à partir de l'analyse par intrusion de mercure réalisée sur l'adsorbant et représentée par la distribution en volume $d$V/$d$logD$_{Hg}$, en fonction du diamètre apparent des pores D$_{Hg}$ (cf. Figure 1). La distribution présente un pic distinct dans le domaine des macropores correspondant à une distribution unimodale autour d'un mode égal à environ 360 nm.

La teneur en phase non zéolithique est égal à 5% poids, tel que mesuré par DRX, en utilisant comme référence, les cristaux de la zéolithe de départ ayant subi le même échange au baryum.

On mesure également la résistance mécanique selon la méthode présentée dans la description de l'invention. La pression nécessaire pour obtenir 0,5% de fines est de 2,5 MPa.

**Exemple 3 :** (comparatif)

***Préparation d'un adsorbant zéolithique sous forme de concassés avec une zéolithe de type XPH, taille des cristaux de zéolithe de 4,5 $\mu$m et un liant de type kaolin non zéolithisé.***

Des granulés (200 g) obtenus à partir de la poudre synthétisée dans l'exemple A sont échangés au moyen d'une solution de chlorure de baryum 0,7 M à 95°C en 4 étapes. À chaque étape, le ratio volume de solution sur masse de solide est de 20 mL.g$^{-1}$ et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 heures sous courant d'azote.

Le taux d'échange en baryum est de 97% et la perte au feu est de 5,5%. Le volume microporeux et la surface externe mesurés selon la méthode du t-plot à partir de l'isotherme d'adsorption d'azote à 77 K après dégazage sous vide à 400°C pendant 10 heures sont respectivement 0,192 cm$^3$.g$^{-1}$ et 70 m$^2$.g$^{-1}$.

Le volume total des macropores et mésopores mesuré par porosimétrie mercure est de 0,33 cm$^3$.g$^{-1}$. La fraction de volume des macropores sur le volume total des macropores et mésopores est égale à 0,6.

On mesure également la résistance mécanique selon la méthode présentée dans la description de l'invention. La pression nécessaire pour obtenir 0,5% de fines est de 2,1 MPa.

La distribution de diamètre de pores est déterminée à partir de l'analyse par intrusion de mercure réalisée sur l'adsorbant et représentée par la distribution en volume $d$V/$d$logD$_{Hg}$, en fonction du diamètre apparent des pores D$_{Hg}$ en Figure 1. La distribution présente un pic et un épaulement dans le domaine des macropores, décrivant une distribution bimodale avec un premier mode aux alentours de 140 nm et un second dans les domaines des très petits macropores, à environ 55 nm.

**Exemple 4** (selon l'invention) :

***Préparation d'un adsorbant zéolithique sous forme de concassés avec cristaux de type XPH de taille 4,5 $\mu$m et un liant de type kaolin zéolithisé***

Des granulés (200 g) obtenus à partir de la poudre synthétisée dans l'exemple A sont placés dans un réacteur en verre muni d'une double enveloppe régulée à une température de 100°C $\pm$ 1°C, puis on ajoute 1,5 L d'une solution aqueuse d'hydroxyde de sodium de concentration 1 M et on laisse le milieu réactionnel sous agitation pendant une durée de 3 heures.

On procède ensuite au lavage des agglomérés en 3 opérations successives de lavage à l'eau suivi de la vidange du réacteur. On s'assure de l'efficacité du lavage en mesurant le pH final des eaux de lavage compris entre 10,0 et 10,5.

Ces agglomérés sont échangés au moyen d'une solution de chlorure de baryum 0,5 M à 95°C en 4 étapes. À chaque étape, le ratio volume de solution sur masse de solide est de 20 mL.g$^{-1}$ et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 heures sous courant d'azote.

Le taux d'échange en baryum est de 96% et la perte au feu est de 5,3%. Le volume microporeux et la surface externe mesurés selon la méthode du t-plot à partir de l'isotherme d'adsorption d'azote à 77 K après dégazage sous vide à 400°C pendant 10 heures sont respectivement 0,260 cm$^3$.g$^{-1}$ et 12 m$^2$.g$^{-1}$.

Le volume total des macropores et des mésopores mesuré par porosimétrie mercure est de 0,29 cm$^3$.g$^{-1}$. La fraction de volume des macropores sur le volume total des macropores et mésopores est égale à 0,9.

La distribution de diamètre de pores est déterminée à partir de l'analyse par intrusion de mercure réalisée sur l'adsorbant et représentée par la distribution en volume $d$V/$d$logD$_{Hg}$, en fonction du diamètre apparent des pores D$_{Hg}$ en Figure 1. La distribution présente un pic distinct dans le domaine des macropores correspondant à une distribution unimodale autour d'un mode égal à environ 190 nm.

La Figure 1 représente les courbes de distributions de diamètre de pores déterminées à partir de l'analyse par intrusion de mercure réalisée sur les adsorbants des exemples 2 à 4.

La teneur en phase non zéolithique est égal à 5% poids, tel que mesuré par DRX, en utilisant comme référence, les cristaux de la zéolithe de départ ayant subi le même échange au baryum.

On mesure également la résistance mécanique selon la méthode présentée dans la description de l'invention. La pression nécessaire pour obtenir 0,5% de fines est de 2,5 MPa.

**Exemple 5 :**

Un test de perçage (chromatographie frontale) est ensuite réalisé sur ces adsorbants pour évaluer leur efficacité. La quantité d'adsorbant utilisée pour ce test est d'environ 34 g.

Le mode opératoire pour obtenir les courbes de perçage est le suivant :

- remplissage de la colonne par le tamis et mise en place dans le banc de test,
- remplissage par le solvant de désorption à température ambiante,
- montée progressive à la température d'adsorption sous flux de solvant (5 cm$^3$/min),
- injection de solvant à 30 cm$^3$/min lorsque la température d'adsorption est atteinte,
- permutation solvant/charge pour injecter la charge (30 cm$^3$.min$^{-1}$),
- l'injection de la charge est ensuite maintenue un temps suffisant pour atteindre l'équilibre thermodynamique (c'est-à-dire jusqu'à ce que la concentration en solvant dans l'effluent soit nulle), et
- collecte et analyse de l'effluent du perçage.

Le solvant de désorption utilisé est le para-diéthylbenzène. La composition de la charge est la suivante :

- Paraxylène : 45% poids
- Métaxylène : 45% poids
- Iso-octane: 10% poids (celui-ci est utilisé comme traceur pour l'estimation des volumes non-sélectifs et n'intervient pas dans la séparation)

Le test est réalisé avec une température d'adsorption de 175°C. La pression est suffisante pour que la charge reste en phase liquide, soit 1,5 MPa. La vitesse superficielle (débit/section de la colonne) de circulation du liquide à la température du test est d'environ 1,3 cm.s$^{-1}$ pour l'ensemble des tests.

La sélectivité du paraxylène par rapport au métaxylène est calculée par bilan matière. Les résultats du perçage sont consignés dans le Tableau 1 ci-dessous :

-- Tableau 1 --

| Adsorbant | sélectivité PY/MX | Capacité d'adsorption (%) | HEPT (%) | IC | Résistance mécanique |
|---|---|---|---|---|---|
| Exemple 1 (comparatif) | 3,35 | 14,2 | 6,3 | 7,6 | 2,2 |
| Exemple 2 (comparatif) | 3,52 | 17,4 | 8,4 | 7,3 | 2,5 |
| Exemple 3 (comparatif) | 2,66 | 13,4 | 2,6 | 13,7 | 2,1 |

(suite)

| Adsorbant | sélectivité PX/MX | Capacité d'adsorption (%) | HEPT (%) | IC | Résistance mécanique |
|---|---|---|---|---|---|
| Exemple 4 (invention) | 3,19 | 17,6 | 3,7 | 15,2 | 2,5 |

Légende
- PX = ParaXylène ; MX = MétaXylène
- Capacité d'adsorption exprimée en % ($cm^3$ de $C_8$-aromatiques adsorbés par $cm^3$ de colonne)
- HEPT = Hauteur Équivalente de Plateaux Théoriques mesuré sur le paraxylène exprimée en % de longueur de colonne

$$IC = \frac{\text{sélectivité x capacité}}{\text{HEPT}}$$

Par rapport aux résultats obtenus avec l'adsorbant des exemples 1 et 2, on constate une nette amélioration du transfert de matière sur l'adsorbant de l'exemple 4, traduite par la hauteur équivalente de plateaux théoriques considérablement diminuée.

Par rapport aux résultats obtenus avec l'adsorbant de l'exemple 3, on constate, pour l'adsorbant de l'exemple 4, une nette augmentation de la sélectivité du paraxylène vis-à-vis du métaxylène (+17%) et une nette augmentation de la capacité d'adsorption.

L'indice IC combinant l'ensemble de ces paramètres, capacité, sélectivité et HEPT, permet d'évaluer l'impact du compromis entre sélectivité, capacité et transfert de matière : plus l'indice est élevé, meilleur est le compromis. On note que les indices IC calculés sur les adsorbants à base de cristaux de XPH, à savoir sur les adsorbants des exemples 3 et 4 (exemple 4 étant selon l'invention) sont très nettement supérieurs aux indices calculés sur les adsorbants des exemples 1 et 2.

L'indice IC calculé le plus élevé est obtenu sur l'adsorbant de l'exemple 4, selon l'invention, et par conséquent cet adsorbant sera le plus performant pour la séparation du paraxylène.

L'adsorbant zéolithique selon l'invention allie une bonne résistance mécanique, une bonne sélectivité d'adsorption du paraxylène, une capacité d'adsorption élevée et un transport des molécules au sein de l'adsorbant rapide.

## Revendications

1. Adsorbant comprenant une phase zéolithique et une phase non zéolithique, ledit adsorbant présentant :

   - une surface externe inférieure ou égale à 30 $m^2.g^{-1}$, de préférence inférieure ou égale à 20 $m^2.g^{-1}$, calculée par la méthode du t-plot à partir de l'isotherme d'adsorption d'azote à une température de 77K, après dégazage sous vide (P < $6,7.10^{-4}$ Pa), à une température comprise entre 300°C et 450°C pendant une durée allant de 9 heures à 16 heures,
   - une phase zéolithique comprenant au moins une zéolithe de structure FAU de type X,
   - et une distribution de diamètre de pores, déterminée par intrusion de mercure selon la norme ASTM D4284-83 et exprimée par la distribution en volume $dV/d$log$D_{Hg}$, où $D_{Hg}$ est le diamètre apparent des pores, et V, le volume poreux, dont le mode est compris entre 100 nm et 250 nm, bornes incluses.

2. Adsorbant selon la revendication 1, dont le volume microporeux, évalué par la méthode de t-plot à partir de l'isotherme d'adsorption d'azote ($N_2$) à une température de 77K, est supérieur à 0,200 $cm^3.g^{-1}$, de préférence compris entre 0,205 $cm^3.g^{-1}$ et 0,300 $cm^3.g^{-1}$ et de préférence encore compris entre 0,205 $cm^3.g^{-1}$ et 0,290 $cm^3.g^{-1}$.

3. Adsorbant selon l'une quelconque des revendications précédentes comprenant une phase non zéolithique dans une teneur comprise entre 2% et 8% poids par rapport au poids total de l'adsorbant.

4. Adsorbant selon l'une quelconque des revendications précédentes comprenant du baryum ou du baryum et du potassium.

5. Adsorbant selon l'une quelconque des revendications précédentes présentant un volume total contenu dans les macropores et les mésopores (somme du volume macroporeux et du volume mésoporeux) mesuré par intrusion de mercure selon la norme ASTM D4284-83, compris entre 0,15 cm$^3$.g$^{-1}$ et 0,5 cm$^3$.g$^{-1}$ bornes incluses.

6. Adsorbant selon l'une quelconque des revendications précédentes présentant un ratio (volume macroporeux)/(volume macroporeux + volume mésoporeux) compris entre 0,2 et 1 bornes incluses.

7. Adsorbant selon l'une quelconque des revendications précédentes présentant un rapport atomique Si/Al compris entre 1,00 et 1,50, bornes incluses.

8. Procédé de préparation de l'adsorbant selon les revendications 1 à 7, comprenant au moins les étapes de :

   a) agglomération de cristaux d'au moins une zéolithe FAU de type X, présentant une surface externe, mesurée par adsorption d'azote, supérieure à 20 m$^2$.g$^{-1}$, de préférence comprise entre 20 m$^2$.g$^{-1}$ et 200 m$^2$.g$^{-1}$, bornes incluses, de préférence encore comprise entre 40 m$^2$.g$^{-1}$ et 150 m$^2$.g$^{-1}$, bornes incluses, calculée par la méthode du t-plot à partir de l'isotherme d'adsorption d'azote à une température de 77K, après dégazage sous vide (P < 6,7.10$^{-4}$ Pa), à une température comprise entre 300°C et 450°C pendant une durée allant de 9 heures à 16 heures, avec un liant comprenant de préférence au moins 80% d'argile ou d'un mélange d'argiles et avec jusqu'à 5% d'additifs ainsi qu'avec la quantité d'eau qui permet la mise en forme du matériau aggloméré, puis séchage et calcination des agglomérats ;
   b) zéolithisation d'une partie du liant par mise en contact des agglomérats obtenus à l'étape a) avec une solution basique aqueuse, éventuellement en présence d'au moins un agent structurant ;
   c) élimination optionnelle de l'agent structurant éventuellement présent ;
   d) échange(s) cationique(s) des agglomérats de l'étape b) ou c) par mise en contact avec une solution d'ions baryum ou d'ions baryum et d'ions potassium ;
   e) échange cationique éventuel supplémentaire des agglomérats de l'étape d) par mise en contact avec une solution d'ions potassium ;
   f) lavage et séchage des agglomérats obtenus aux étapes d) ou e), à une température comprise entre 50°C et 150°C ; et
   g) obtention de l'adsorbant zéolithique selon l'invention par activation des agglomérats obtenus à l'étape f) sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température comprise entre 100°C et 400°C, de préférence entre 200°C et 300°C.

9. Procédé selon la revendication 8, dans lequel le liant d'agglomération mis en œuvre à l'étape a) contient au moins une argile zéolithisable, de préférence choisie dans la famille des kaolins, kaolinites, nacrites, dickites, halloysites et métakaolins, et leurs mélanges.

10. Procédé selon la revendication 8 ou 9, dans lequel les cristaux de zéolithe FAU utilisés lors de l'étape d'agglomération (étape a) présentent un diamètre moyen en nombre compris entre 1 μm et 20 μm, bornes incluses, de préférence encore compris entre 1,5 μm et 20 μm, bornes incluses, plus préférentiellement entre 1,8 μm et 10 μm, bornes incluses, mieux encore entre 2 μm et 10 μm, bornes incluses, et de préférence encore entre 2 μm et 8 μm, bornes incluses.

11. Procédé selon l'une quelconque des revendications 8 à 10, dans lequel la zéolithe FAU utilisée à l'étape a) est une zéolithe FAU à porosité hiérarchisée.

12. Utilisation d'un adsorbant selon l'une quelconque des revendications 1 à 7 ou préparé selon l'une des revendications 8 à 11, comme agent d'adsorption dans :

   • la séparation de coupes d'isomères aromatiques en C8 et notamment des xylènes,
   • la séparation d'isomères de toluène substitué tels que nitrotoluène, diéthyltoluène, toluènediamine, et autres,
   • la séparation des crésols,
   • la séparation des alcools polyhydriques, tels que les sucres.

13. Procédé de séparation des isomères de xylènes en phase gaz ou en phase liquide mettant en œuvre au moins un adsorbant selon l'une quelconque des revendications 1 à 7 ou préparé selon l'une des revendications 8 à 11.

14. Procédé de séparation selon la revendication 13, qui est un procédé de séparation de paraxylène à partir d'une charge à traiter de coupes d'isomères aromatiques à 8 atomes de carbone, utilisant, comme agent d'adsorption du paraxylène, un adsorbant selon l'une quelconque des revendications 1 à 7 ou préparé selon l'une des revendications 8 à 11.

15. Procédé selon la revendication 14 mis en œuvre dans une unité d'adsorption en lit mobile simulé à contre-courant simulé dans les conditions opératoires :

• nombre de lits : 4 à 24 ;
• nombre de zones : au moins 4 zones de fonctionnement, chacune étant localisée entre un point d'alimentation et un point de soutirage;
• température comprise entre 100 à 250°C ;
• pression comprise entre la pression de bulle des xylènes (ou du toluène lorsque le toluène est choisi comme désorbant) à la température du procédé et 3 MPa ;
• rapport des débits désorbant sur charge à traiter : 0,7 à 2,5 ;
• taux de recyclage compris entre 2 et 12 de préférence entre 2,5 et 6,
• temps de cycle, correspondant au temps entre deux injections de désorbant sur un lit donné : compris entre 4 et 25 min.

16. Procédé selon la revendication 15, dans lequel le désorbant est le toluène ou le *para*-diéthylbenzène.

17. Procédé selon la revendication 15 ou la revendication 16, dans lequel la teneur en eau dans les flux entrants constitués par les flux de charge et/ou de désorbant est ajustée entre 20 ppm et 150 ppm.

**Patentansprüche**

1. Adsorbens, umfassend eine Zeolithphase und eine Nicht-Zeolithphase, wobei das Adsorbens aufweist:

- eine äußere Oberfläche kleiner oder gleich 30 $m^2.g^{-1}$, vorzugsweise kleiner oder gleich 20 $m^2.g^{-1}$, berechnet anhand der t-plot-Methode auf der Basis der Adsorptionsisotherme von Stickstoff bei einer Temperatur von 77 K, nach Entgasung unter Vakuum (P < $6,7.10^{-4}$ Pa) bei einer Temperatur zwischen 300 °C und 450 °C während einer Dauer von 9 Stunden bis 16 Stunden,
- eine Zeolithphase, die mindestens einen Zeolith mit FAU-Struktur vom Typ X umfasst,
- und eine Porendurchmesserverteilung, bestimmt durch Quecksilberintrusion gemäß der Norm ASTM D4284-83 und ausgedrückt durch die Volumenverteilung $dV/d\log D_{Hg}$, wobei $D_{Hg}$ der scheinbare Durchmesser der Poren und V das poröse Volumen ist, dessen Modus zwischen 100 nm und 250 nm, Grenzen inbegriffen, liegt.

2. Adsorbens nach Anspruch 1, dessen mikroporöses Volumen, beurteilt anhand der t-plot-Methode auf der Basis der Adsorptionsisotherme von Stickstoff ($N_2$) bei einer Temperatur von 77 K, größer als 0,200 $cm^3.g^{-1}$ ist, vorzugsweise zwischen 0,205 $cm^3.g^{-1}$ und 0,300 $cm^3.g^{-1}$ und vorzugsweise noch zwischen 0,205 $cm^3.g^{-1}$ und 0,290 $cm^3.g^{-1}$ liegt.

3. Adsorbens nach einem der vorangehenden Ansprüche, umfassend eine Nicht-Zeolithphase in einem Gehalt, der zwischen 2 % und 8 % Gewicht in Bezug auf das Gesamtgewicht des Adsorbens liegt.

4. Adsorbens nach einem der vorangehenden Ansprüche, umfassend Barium oder Barium und Kalium.

5. Adsorbens nach einem der vorangehenden Ansprüche, aufweisend ein Gesamtvolumen, enthalten in den Makroporen und den Mesoporen (Summe des makroporösen Volumens und des mesoporösen Volumens), gemessen durch Quecksilberintrusion gemäß der Norm ASTM D4284-83, das zwischen 0,15 $cm^3.g^{-1}$ und 0,5 $cm^3.g^{-1}$, Grenzwerte inbegriffen, liegt.

6. Adsorbens nach einem der vorangehenden Ansprüche, aufweisend eine Quote (makroporöses Volumen)/(makroporöses Volumen + mesoporöses Volumen), die zwischen 0,2 und 1, Grenzwerte inbegriffen, liegt.

7. Adsorbens nach einem der vorangehenden Ansprüche, aufweisend ein Atomverhältnis Si/Al, das zwischen 1,00 und 1,50, Grenzwerte inbegriffen, liegt.

8. Verfahren zur Herstellung des Adsorbens nach den Ansprüchen 1 bis 7, umfassend mindestens die folgenden Schritte:

a) Agglomeration von Kristallen von mindestens einem FAU-Zeolith vom Typ X, aufweisend eine äußere Oberfläche, gemessen durch Stickstoffadsorption, größer als 20 $m^2.g^{-1}$, vorzugsweise zwischen 20 $m^2.g^{-1}$ und 200 $m^2.g^{-1}$, Grenzwerte inbegriffen, vorzugsweise noch zwischen 40 $m^2.g^{-1}$ und 150 $m^2.g^{-1}$, Grenzwerte inbegriffen, berechnet anhand der t-plot-Methode auf der Basis der Adsorptionsisotherme von Stickstoff bei einer Temperatur von 77 K, nach Entgasung unter Vakuum (P < 6,7.$10^{-4}$ Pa) bei einer Temperatur zwischen 300 °C und 450 °C während einer Dauer von 9 Stunden bis 16 Stunden, mit einem Bindemittel, umfassend vorzugsweise mindestens 80 % Ton oder eines Tongemischs und mit bis zu 5 % Additiven sowie mit der Wassermenge, die das Formen des agglomerierten Materials erlaubt, dann Trocknen und Calcinieren der Agglomerate;

b) Zeolithisieren eines Teils des Bindemittels durch Inkontaktversetzen der in Schritt a) erhaltenen Agglomerate mit einer wässrigen basischen Lösung, eventuell bei Anwesenheit von mindestens einem strukturierenden Mittel;

c) optionales Entfernen des eventuell vorhandenen strukturierenden Mittels;

d) Kationenaustausch(e) der Agglomerate von Schritt b) oder c) durch Inkontaktversetzen mit einer Bariumionen- oder Bariumionen- und Kaliumionenlösung;

e) eventuell zusätzlicher Kationenaustausch der Agglomerate von Schritt d) durch Inkontaktversetzen mit einer Kaliumionenlösung;

f) Waschen und Trocken der in den Schritten d) oder e) erhaltenen Agglomerate bei einer Temperatur zwischen 50 °C und 150 °C; und

g) Erhalten des erfindungsgemäßen Zeolithadsorbens durch Aktivierung der in Schritt f) erhaltenen Agglomerate mit Oxidations- und/oder Inertgasspülen mit insbesondere solchen Gasen wie Sauerstoff, Stickstoff, Luft, einer trockenen und/oder decarbonierten Luft, einer sauerstoffabgereicherten Luft, eventuell trocken und/oder decarboniert, bei einer Temperatur zwischen 100 °C und 400 °C, vorzugsweise zwischen 200 °C und 300 °C.

9. Verfahren nach Anspruch 8, wobei das in Schritt a) eingesetzte Agglomerationsbindemittel mindestens einen zeolithisierbaren Ton enthält, der vorzugsweise aus der Familie der Kaoline, Kaolinite, Nacrite, Dickite, Halloysite und Metakaoline und deren Gemischen ausgewählt ist.

10. Verfahren nach Anspruch 8 oder 9, wobei die beim Agglomerationsschritt (Schritt a) verwendeten FAU-Zeolithkristalle einen zahlenmittleren Durchmesser zwischen 1 $\mu$m und 20 $\mu$m, Grenzwerte inbegriffen, vorzugsweise noch zwischen 1,5 $\mu$m und 20 $\mu$m, Grenzwerte inbegriffen, vorzugsweiser zwischen 1,8 $\mu$m und 10 $\mu$m, Grenzwerte inbegriffen, besser noch zwischen 2 $\mu$m und 10 $\mu$m, Grenzwerte inbegriffen, und vorzugsweise noch zwischen 2 $\mu$m und 8 $\mu$m, Grenzwerte inbegriffen, aufweisen.

11. Verfahren nach einem der Ansprüche 8 bis 10, wobei der in Schritt a) eingesetzte FAU-Zeolith ein FAU-Zeolith mit hierarchisierter Porosität ist.

12. Verwendung eines Adsorbens nach einem der Ansprüche 1 bis 7 oder hergestellt nach einem der Ansprüche 8 bis 11 als Adsorptionsmittel bei:

• der Trennung von aromatischen C8-Isomerfraktionen und insbesondere der Xylene,
• der Trennung von Isomeren substituierten Toluens wie Nitrotoluen, Diethyltoluen, Toluendiamin und anderer,
• der Trennung der Cresole,
• der Trennung der mehrwertigen Alkohole wie Zucker.

13. Verfahren zum Trennen der Xylenisomere in Gasphase oder in flüssiger Phase, bei dem mindestens ein Adsorbens nach einem der Ansprüche 1 bis 7 oder hergestellt nach einem der Ansprüche 8 bis 11 zum Einsatz kommt.

14. Trennverfahren nach Anspruch 13, das ein Paraxylentrennverfahren auf der Basis einer zu behandelnden Charge aromatischer Isomerfraktionen mit 8 Kohlenstoffatomen ist, das als Adsorptionsmittel des Paraxylens ein Adsorbens nach einem der Ansprüche 1 bis 7 oder hergestellt nach einem der Ansprüche 8 bis 11 verwendet.

15. Verfahren nach Anspruch 14, durchgeführt in einer Adsorptionseinheit mit simuliertem mobilem Bett mit simuliertem Gegenstrom unter den operativen Bedingungen:

• Anzahl der Betten: 4 bis 24;
• Anzahl der Zonen: mindestens 4 Betriebszonen, wobei jede zwischen einem Versorgungspunkt und einem Entnahmepunkt lokalisiert ist;

• Temperatur zwischen 100 bis 250 °C;
• Druck zwischen dem Blasendruck der Xylene (oder des Toluens, wenn Toluen als Desorptionsmittel gewählt ist) bei Verfahrenstemperatur und 3 MPa;
• Verhältnis der Desorptionsmittelmengen zu der zu behandelnden Charge: 0,7 bis 2,5;
• Recyclinggrad zwischen 2 und 12, vorzugsweise zwischen 2,5 und 6,
• Zykluszeit, die der Zeit zwischen zwei Einleitungen von Desorptionsmittel auf ein bestimmtes Bett entspricht: zwischen 4 und 25 Min.

16. Verfahren nach Anspruch 15, wobei das Desorptionsmittel Toluen oder Para-diethylbenzol ist.

17. Verfahren nach Anspruch 15 oder Anspruch 16, wobei der Wassergehalt in den eintretenden Strömen, gebildet von den Chargenströmen und/oder dem Desorptionsmittel, auf zwischen 20 ppm und 150 ppm eingestellt wird.

**Claims**

1. An adsorbent comprising a zeolite-based phase and a non-zeolite-based phase, wherein said adsorbent presents:

   - an outer surface area of less than or equal to 30 $m^2.g^{-1}$, preferably of less than or equal to 20 $m^2.g^{-1}$, calculated via the t-plot method from the nitrogen adsorption isotherm at a temperature of 77 K, after degassing under vacuum (P<$6.7.10^{-4}$ Pa), at a temperature of between 300°C and 450°C for a time ranging from 9 hours to 16 hours,
   - a zeolite-based phase comprising at least one zeolite of FAU structure of X type,
   - and a pore diameter distribution, determined by mercury intrusion according to standard ASTM D 4284-83 and expressed by the volume distribution dV/dlog $D_{Hg}$, wherein $D_{Hg}$ is the apparent pore diameter and V is the pore volume, the mode of which is between 100 nm and 250 nm, limits inclusive.

2. The adsorbent according to claim 1, wherein the micropore volume, evaluated via the t-plot method from the nitrogen ($N_2$) adsorption isotherm at a temperature of 77 K, greater than 0.200 $cm^3g^{-1}$, preferably comprised between 0.205 $cm^3.g^{-1}$ and 0.300 $cm^3.g^{-1}$ and still preferably comprised between 0.205 $cm^3.g^{-1}$ and 0.290 $cm^3.g^{-1}$.

3. The adsorbent according to any one of the preceding claims, comprising a non-zeolite-based phase at a content of between 2% and 8% by weight relative to the total weight of the adsorbent.

4. The adsorbent according to any one of the preceding claims, comprising barium or barium and potassium.

5. The adsorbent according to any one of the preceding claims, having a total volume contained in the macropores and the mesopores (sum of the macropore and mesopore volume) measured by mercury intrusion according to standard ASTM D4284-83 of between 0.15 $cm^3.g^{-1}$ and 0.5 $cm^3.g^{-1}$, limits inclusive.

6. The adsorbent according to any one of the preceding claims, having a (macropore volume)/(macropore volume+mesopore volume) ratio of between 0.2 and 1, limits inclusive.

7. The adsorbent according to any one of the preceding claims, having an Si/Al atomic ratio of between 1.00 and 1.50, limits inclusive.

8. A process for preparing the adsorbent according to claims 1 to 7, comprising at least the steps of:

   a) agglomeration of crystals of at least one FAU zeolite having an outer surface area as measured by nitrogen adsorption, of greater than 20 $m^2.g^{-1}$, preferably comprised between 20 $m^2.g^{-1}$ and 200 $m^2.g^{-1}$, more preferably comprised between 40 $m^2.g^{-1}$ and 150 $m^2.g^{-1}$, limits inclusive, calculated via the t-plot method from the nitrogen adsorption isotherm at a temperature of 77 K, after degassing under vacuum (P<$6.7.10^{-4}$ Pa), at a temperature of between 300°C and 450°C for a time ranging from 9 hours to 16 hours, with a binder preferably comprising at least 80 % of clay or of a mixture of clays and with up to 5% of additives and also with an amount of water which allows the forming of an agglomerated material, followed by drying and calcination of the agglomerates;
   b) zeolitization of one part of the binder by placing the agglomerates obtained in step a) in contact with an aqueous basic solution, optionally in the presence of at least one structuring agent;
   c) optionally, removal of the structuring agent optionally present;

d) cationic exchange(s) of the agglomerates of step b) or c) by placing in contact with a solution of barium ions or of barium ions and potassium ions;

e) optionally, carrying out additional cationic exchange of the agglomerates of step d) by placing in contact with a solution of potassium ions;

f) washing and drying of the agglomerates obtained in step d) or e), at a temperature of between 50°C and 150°C; and

g) producing the zeolite-based adsorbent according to the invention by activating the agglomerates obtained in step f) under a stream of oxidizing and/or inert gas, especially with gases such as oxygen, nitrogen, air, a dry and/or decarbonated air, or an oxygen-depleted air, which is optionally dry and/or decarbonated, at a temperature of between 100°C and 400°C, preferably between 200°C and 300°C.

9. The process according to claim 8, wherein the agglomeration binder used in step a) contains at least one zeolitizable clay, preferably chosen from the family of kaolins, kaolinites, nacrites, dickites, halloysites and metakaolins, and mixtures thereof.

10. The process according to claim 8 or 9, wherein the FAU zeolite crystals used during the agglomeration step (step a) have a number-mean diameter of between 1 μm and 20 μm, limits inclusive, more preferably between 1.5 μm and 20 μm, limits inclusive, more preferentially between 1.8 μm and 10 μm, limits inclusive, better still between 2 μm and 10 μm, limits inclusive, and more preferably between 2 μm and 8 μm, limits inclusive.

11. The process according to any one of claims 8 to 10, wherein the FAU zeolite used in step a) is a hierarchically porous FAU zeolite.

12. Use of an adsorbent according to claims 1 to 7 or prepared according to any one of claims 8 to 11, as an adsorption agent in:

   • the separation of C8 aromatic isomer fractions and especially of xylenes,
   • the separation of substituted toluene isomers such as nitrotoluene, diethyltoluene, toluenediamine, and the like,
   • the separation of cresols,
   • the separation of polyhydric alcohols, such as sugars.

13. A process for the gas-phase or liquid-phase separation of xylene isomers using at least one adsorbent according to any one of claims 1 to 7 or prepared according to any one of claims 8 to 11.

14. A process according to claim 13, which is a process for separating para-xylene from a feedstock of aromatic isomer fractions containing 8 carbon atoms, using, as para-xylene adsorption agent, an adsorbent according to any one of claims 1 to 7 or prepared according to any one of claims 8 to 11.

15. The process according to claim 14, carried out in a counter-current simulated moving bed adsorption unit, under the following operating conditions:

   • number of beds: 4 to 24;
   • number of zones: at least 4 operating zones, each being located between a feed point and a withdrawal point;
   • temperature between 100°C and 250°C;
   • pressure between the bubble pressure of xylenes (or of toluene when toluene is chosen as desorbent) at the process temperature and 3 MPa;
   • ratio of the flow rates of desorbent to feedstock to be treated: 0.7 to 2.5;
   • recycling rate between 2 and 12, preferably between 2.5 and 6 ;
   • cycle time, corresponding to the time between two injections of desorbent onto a given bed: between 4 and 25 minutes.

16. The process according to claim 15, wherein the desorbent is toluene or para-diethylbenzene.

17. The process according to claim 15 or claim 16 wherein a water content in the inlet streams constituted by the feedstock and/or desorbent streams is adjusted to between 20 ppm and 150 ppm.

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- FR 2789914
- US 8283274 B
- CN 1267185
- US 20090326308 A
- US 7785563 B
- WO 2013106816 A
- WO 2007043731 A
- US 2985589 A
- US 5284992 A
- US 5629467 A
- US 4402832 A
- US 4498991 A

**Littérature non-brevet citée dans la description**

- Préparation of granular X-type zeolite/activated carbon composite from elutrilithe by adding pitch and solid SiO2. *Materials Chemistry and Physics,* 15 Juillet 2014, vol. 147 (3), 1003-1008
- « Principles of Adsorption and Adsorption Processes » (« Principes de l'Adsorption et des Procédés d'Adsorption »). John Wiley & Sons, 1984, 326-407
- **RUTHVEN.** *Principles of Adsorption and Adsorption Processes,* 248-250
- **INAYAT.** *Angew. Chem. Int. Ed.,* 2012, vol. 51, 1962-1965
- *Adv. Funct. Mater.,* 2012, vol. 22, 916-928
- **RUTHVEN.** Principles of Adsorption and Adsorption Processes. John Wiley & Sons, 1984
- *Angew. Chem. Int. Ed.,* 2012, vol. 51, 1962-1965